# EUROPEAN PATENT SPECIFICATION

(11) **EP 4 322 968 B1**
(45) Date of publication and mention of the grant of the patent: **19.03.2025**
(21) Application number: 22722530.7
(22) Date of filing: 13.04.2022
(51) Int. Cl.: A61K 33/244, A61K 9/00, A61K 9/10, A61K 9/14, A61K 47/02, A61K 47/10, A61K 47/18, A61K 47/38, A61P 27/00, A61P 27/06, A61P 35/00

(54) **OPHTHALMIC TOPICAL COMPOSITION WITH CERIA NANOPARTICLES FOR TREATING DISEASES OF POSTERIOR SEGMENT OF THE EYE**
OPHTHALMISCHE TOPISCHE ZUSAMMENSETZUNG MIT CEROXID-NANOTEILCHEN ZUR BEHANDLUNG VON ERKRANKUNGEN DES HINTEREN AUGENSEGMENTS
COMPOSITION TOPIQUE OPHTALMIQUE AVEC DES NANOPARTICULES À CÉRIUM POUR LE TRAITEMENT DES MALADIES DU SEGMENT POSTÉRIEUR DE L'OEIL

(30) Priority: 15.04.2021 EP 21382320
(43) Date of publication of application: 21.02.2024
(73) Proprietor: Fundació Hospital Universitari Vall d'Hebron - Institut de Recerca, 08035 Barcelona (ES); Fundació Institut Català de Nanociència i Nanotecnologia (ICN2), 08193 Bellaterra (ES); Institució Catalana De Recerca I Estudis Avançats (ICREA), 08010 Barcelona (ES)
(72) Inventor: FRANCO PUNTES, Víctor, 08028 BARCELONA (ES); CASALS MERCADAL, Eudald, 08210 BARBERÀ DEL VALLÈS (ES); ZAPATA VICTORI, Miguel Angel, 08173 SANT CUGAT DEL VALLÈS (ES); SALAS TORRAS, Anna, 08203 SABADELL (ES); DUARRI PIQUÉ, Anna, 08241 MANRESA (ES); BADIA PÉREZ, Anna, 08230 MATADEPERA (ES); GARCÍA ARUMÍ, Josep, 08017 BARCELONA (ES)
(74) Representative: ZBM Patents - Zea, Barlocci & Markvardsen
(86) International application number: PCT/EP2022/059891
(87) International publication number: WO 2022/219050

(56) References cited:
- WO-A1-2017/174437
- WO-A2-2008/064357
- US-A1- 2010 221 344
- BADIA ANNA ET AL: "Firsts steps in the development of a topical CeO2NPs treatment to fight dry AMD progression using the DKOrd8 mouse model", MEETING INFO: 2018 ANNUAL MEETING OF THE ASSOCIATION FOR RESEARCH IN VISION AND OPHTHALMOLOGY, ARVO 2018, 29 April 2018 (2018-04-29), XP055838006, Retrieved from the Internet <URL:https://iovs.arvojournals.org/article.aspx?articleid=2688980> [retrieved on 20210906]
- TISI ANNAMARIA ET AL: "Nanoceria Particles Are an Eligible Candidate to Prevent Age-Related Macular Degeneration by Inhibiting Retinal Pigment Epithelium Cell Death and Autophagy Alterations", CELLS, vol. 9, no. 7, 4 July 2020 (2020-07-04), pages 1617, XP055837989, DOI: 10.3390/cells9071617
- HANCOCK MATTHEW L ET AL: "The characterization of purified citrate-coated cerium oxide nanoparticles prepared via hydrothermal synthesis", APPLIED SURFACE SCIENCE, ELSEVIER, AMSTERDAM, NL, vol. 535, 28 August 2020 (2020-08-28), pages 147681, XP086309227, ISSN: 0169-4332, [retrieved on 20200828], DOI: 10.1016/J.APSUSC.2020.147681
- RAZAVI M S ET AL: "Recent Developments of Nanostructures for the Ocular Delivery of Natural Compounds", FRONTIERS IN CHEMISTRY, vol. 10, 13 April 2022 (2022-04-13), pages 850757, XP055936693, DOI: 10.3389/fchem.2022.850757

## Description

The present invention relates to the field of medical approaches for ocular diseases of posterior segment of the eye. The invention also relates to ophthalmic compositions.

### BACKGROUND ART

The eye is a very complex organ with different specialized cells and tissues. Local and systemic diseases may affect the different regions of the eye, but the anatomy of the eye and complex physiology of the retina and optic nerve makes challenging the development of efficacious drugs.

The eye is divided into two compartments called the anterior segment (front of the eye) and posterior segment (back of the eye). The anterior segment of the eye refers to the cornea, conjunctiva, anterior sclera (part of sclera that transitions anteriorly to become cornea at the limbus) iris, ciliary body, aqueous humour and the lens. The posterior segment of the eye (also named posterior eye segment) includes the anterior hyaloid membrane and the structures behind this membrane, posterior sclera (part of sclera that transitions posteriorly into optic nerve dural sheet), the vitreous body (including vitreous humour and membrane), retina, macula, choroid and optic nerve. Posterior segment represents the two-thirds of the eye. The leading causes of vision impairment and irreversible blindness are diseases related with the posterior segment of the eye.

Topical formulations (e.g., eye drops) are the least invasive route for ocular drug administration. Topical local administration is the main modality treatment for anterior segment disease. However, ocular barriers for avoiding pathogens to access also hinder drug delivery even to the anterior segment. In addition, blinking and tear film turnover, designed to wash away foreign material and maintain a smooth clear anterior surface, also limits residence time of a drug. Furthermore, access to posterior segment is hindered by a closely packed corneal epithelium and stroma with varying lipophilicity (see Awwad et al., "Principles of Pharmacology in the Eye", British Journal of Pharmacology-2017, vol no. 174 Issue 23, pp.:4205-4223). Further, the direction of aqueous flow in the eye (ciliary body to anterior chamber angle) is against direction of drug delivery via a topical route.

Even in case a drug can reach the posterior segment of the eye, it may be removed from the vitreous cavity through diffusion into the anterior chamber, or by the blood-retinal barrier. All this makes difficult any eye treatment of a disease of the posterior segment by topically administration onto eye surface, and the only local modalities in everyday use for treating diseases of the posterior segment of the eye are intravitreal injection (IVT), laser photocoagulation or periocular injections. This is mainly due because is generally assumed that topical administrations do not reach the posterior segment of the eye (ie. the vitreous and the retina), as declared in Urtti A et al., "Challenges and obstacles of ocular pharmacokinetics and drug delivery". Adv. Drug. Deliv. Rev. 2006, vol. 58, pp. 1131-1135. IVT injections avoid all barriers and results in the greatest bioavailability. However, IVT injections imply several severe side-effects, such as retinal detachment. In addition, this kind of intervention is totally senseless at early stages of many of the disease of posterior segment of eye.

Main posterior segment ophthalmic disorders include pathological neovascularization and ectopic proliferation, atrophy and nerve cell death, inflammation and infection, and detachment. Diseases and conditions commonly associated with these symptoms include macular degeneration (i.e. age-related macular degeneration), diabetic retinopathy, retinopathy of prematurity, retinitis pigmentosa, macular edema, glaucoma, posterior uveitis, endophthalmitis, ocular insult and ocular manifestation of systemic disease such as viral infection, arthritis and rosacea.

Many of these diseases develop through different stages with different symptoms and gravity. At certain early stages of the development of the disease, an aggressive treatment is not recommended, and patients are simply followed-on and/or they receive certain vitamins and antioxidants usually orally (or systemic or by any other route in other occasions) to slow progression of disease, in part caused by oxidative stress. However, more specific treatments conceived for these diseases at these early stages would deprive the patient of advanced lesions.

This is the particular case of aged-related macular degeneration (abbreviated AMD). In this disease, retinal cell death occurs due to an increase of oxidative stress and inflammatory response. AMD is a neurodegenerative and complex disorder with multifactorial etiology affecting retina and that results in the progressive and irreversible loss of central vision. AMD is one of the major causes of blindness, together with diabetic retinopathy. AMD is a disease currently unavoidable that represents a major cause of blindness in people over 50 and affects millions of people worldwide. Moreover, it is a disease orphan of treatment. At early stages no specific treatment is administered, rather than vitamins and antioxidants, as previously indicated. At advanced stages of the disease, AMD is classified in two types: wet AMD and dry AMD. The wet form of macular degeneration is related to abnormal growth of blood vessels in the retina that can leak blood and can cause damage to photoreceptor cells and retinal detachment. Approved drugs for advanced stages of wet AMD include certain antibodies such as bevacizumab and ranibizumab intravitreal injections, which are aimed to slow progression of disease. In dry AMD, characterized by a massive atrophy of retinal cells in the macula, no specific treatment has been found effective and finally approved.

A similar situation can be observed, precisely, with diabetic retinopathy (abbreviated DR), the most common complication of diabetes and that remains the leading cause of blindness among working-age individuals in developed countries. At early stages of DR, neurodegeneration takes place although it is considered a microcirculatory disease (see Simó et al. on behalf of the European Consortium for Early Treatment of Diabetic Retinopathy (EUROCONDOR). "Neurodegeneration is an early event in diabetic retinopathy: therapeutic implications", Br. J. Ophthalmol. - 2012, vol. 96, pp.1285-1290).

This state of "orphan of treatment" for all these diseases of the posterior segment is challenging. First, because the systemic administration of antioxidants finds as drawback the difficulty of reaching the target (i.e., posterior segment) due to the natural blood retinal barrier in eye. Thus, it is usually difficult to slow progression of disease. Second, in advanced stages when intravitreal injections are already administered, they suppose expensive treatments besides the dangerous side-effects associated, and on many occasions (very) poor patient compliance (i.e., people stop going to the doctor and interrupt the treatment because they hate to have injections in their eyes and because it also slows down a bit the degeneration while increasing pain and risks).

About possible drugs to treat disorders or diseases of the posterior segment, cerium oxide nanoparticles (ceria nanoparticles or Ceria NPs) have been disclosed for intravitreal injections to treat glaucoma, age-related macular degeneration (dry and wet forms) and diabetic retinopathy, among other diseases. Thus, in US patent US7347987B2 administration of ceria nanoparticles to inhibit reactive oxygen species (ROS) and promote longevity of retinal neurons is disclosed. Data are performed in rats to which an intravitreal injection of ceria nanoparticles of unknown diameter size and three-dimensional form was administered. Ultrafine particles are mentioned from 1 to 10 nm, and topical application is also mentioned, although no data are provided.

Another document disclosing the use of ceria nanoparticles to reduce, inhibit or reverse the rate of neovascularization or retinal cell degeneration in diseases of posterior segment of the eye in the US patent application with publication number US2011111007. An in vivo assay (Example 2) with a mouse model of AMD is disclosed by administering ceria nanoparticles of unknown size by intravitreal injection. US2011111007 generically disclose ceria nanoparticles with sizes from 1 to 10 nm. Topical application is mentioned, although no data are provided.

Although the documents propose topical application as general mode of administration, no examples are disclosed precisely because it is not the habitual route of administration of drugs to posterior segment.

Indeed, according to recent prior art, new challenges should also be achieved to translate the use of Ceria nanoparticles to clinical practice and improve patients' treatment in the ophthalmic field: the development of water-soluble ceria nanoparticles; and the development of more permeable ceria nanoparticles to the ocular surface to administer them by a less invasive route of administration, rather than intravitreal injections (see Maccarone et al., 2019. Ophtalmic Applications of Cerium Oxide Nanoparticles. Nanoceria in ophthalmology). In order to improve solubility and permeability, functionalization of particles with saccharides is proposed. To increase corneal permeation, although nanosize can facilitate it, PEGylation and liposome transport are proposed, to avoid nanoparticle aggregation and dramatic size increase. Noteworthy is the fact that ultrafine (1-10 nm) ceria nanoparticles tend to aggregate and to form insoluble clusters that cause severe side-effect, such as immune reactions. Thus, independently of the fact that neither US7347987B2 nor US2011111007 shows a topic ophthalmic treatment with any ceria nanoparticles, following author's works and the state of the art, the proposed ultrafine sizes would imply aggregation and solubility problems that would cause adverse effects. This is, moreover, challenging with the majority of current available ceria nanoparticles, that are in the form of discrete aggregates of few tens of nanometers in the best cases, and the most often with a cationic surface charge, since the vitreous humour has porosity of about 10 nanometers made of an anionic mesh, in such a way that cationic and larger nanoparticles get absorbed onto it. So, they have to be forced inside the eye. In addition, what can show beneficial work in acute conditions could not be directly translated for the chronic inflammation associated with the advanced stages of these diseases of the posterior segment of the eye, such as AMD.

Badia et al, disclosed the use of cerium oxide nanoparticles in a model of AMD, in which the intravitreal injections and the topical administration were assayed (Badia et al., "First steps in the development of topical CeO2NPs treatment to fight dry AMD progression using the DKOrd8 mouse model", IOVS-2018, vol. no. 59(9), Abstract 58). The authors concluded that cerium was detected in the retina 24 hours after either the intravitreal injection or the topical administration. However, the particle size was not disclosed, and the document is silent about the distribution of the nanoparticles in the several parts of the anatomy of the eye.

Other documents of the field disclosing assays with ceria nanoparticles are the following.

The document of Tisi et al., "Nanoceria particles are an eligible candidate to prevent age-related macular degeneration by inhibiting retinal pigment epithelium cell death and autophagy alterations", Cells-2020, vol. no. 9, p. 1617, discloses the intravitreal injection of ceria nanoparticles in rats, although the particle size of the nanoparticles is not indicated, the authors conclude that in view of the localization of the nanoparticles in the RPE, they can be conceived as interesting starting point for continuing investigating to finally see if they could be applied in the treatment of AMD patients and to protect the RPE.

The document US2010221344 also discloses using a composition with nanoceria associated with another drug for treating disorders of the posterior segment of the eye, in particular glaucoma. Examples are carried out with nanoparticles of 10-20 nm to inhibit in a tube assay the enzyme human carbonic anhydrase II (hCAII), associated to glaucoma progression.

The patent application WO2018064357 discloses citrated-coated cerium oxide nanoparticles of 3-5 nm for enhancing the survival and viability of cells. The nanoparticles are proposed to be administered intra-ocularly, but not topically to the eye, and they are silent about any disease of the posterior segment of the eye.

Other coated ceria nanoparticles are disclosed for other uses different than the treatment of diseases of the posterior segment of the eye. For example, the patent application WO2017174437 discloses ceria nanoparticles coated with albumin or polyvinylpyrrolidone for use in the treatment of hepatocellular carcinoma.

Finally, the preparation of citrate-coated ceria nanoparticles with a diameter of about 5 nm and their toxicity in rats is disclosed by Hancock et al., "The characterization of purified citrate-coated cerium oxide nanoparticles prepared via hydrothermal synthesis", Applied Surface Sciences-2020, 147681.

Thus, there is still a need in the field of effective treatments for all these diseases of ocular posterior segment with low side effects and to face initial stages of some of the diseases when intravitreal injections are not conceivable.

### SUMMARY OF THE INVENTION

The inventors have found that certain cerium oxide nanoparticles (abbreviated Ceria NPs) that do not aggregate and are in colloidal suspension in an aqueous media comprising a citrate salt, could be applied topically in the eye (i.e., in the cornea or conjunctival fornix or sclera, that is, ophthalmic application to surface of the eye), and they reached the posterior segment of the eye. Ceria NPs homogeneously distributed in the different parts of the eye (anterior segment, lens, retina). This was achieved with the Ceria NPs with adsorbed citrate in their surface or with citrate as coating, without the need of any functionalization with saccharides, PEG, PVP, EDTA or other for the avoidance of aggregation (i.e., avoidance of precipitation to promote "solubilization" or "homogenous suspension"). Moreover, permeation through the several parts of the anterior segment was accomplished without any nanoparticle surface covalent modification by means of hydrophobic or amphiphilic molecules (i.e., PEGylation), or without the need of encapsulating Ceria NPs into supramolecular structures (i.e., liposomes) containing lipids or hydrophobic or amphiphilic polymeric compounds.

Therefore, inventors have surprisingly found a mode to maintain Ceria NPs of low diameter in colloidal suspension that do not aggregate. The nanoparticles were, thus, isolated single nanoparticles in suspension and monodispersed (i.e., >90% of uniformity in size distribution). These particular suspensions could enter the posterior segment from the anterior segment and no particle aggregation was observed, thus avoiding any secondary effect due to the formation of the precipitates.

Moreover, as will be depicted in examples below, the amount of Ceria NPs that reached the posterior segment (retina and different parts of posterior pole) was about 30-50 % of the amount that was finally detected in the several eye parts after being topically administered. These detected amounts achieved effective concentrations for abrogating the evolution of the diseases. Advantageously, no toxicity was observed in tissues where Ceria NPs were retained, and they were neither detected in liver nor in brain. In addition, as will be illustrated in examples below, the concentration reached in the different parts with topical administration was about half of the reached with intravitreal injection. These were therapeutically effective amounts and, thus, for the first time Ceria NPs could be used as an actual substitute of intravitreal injections for the treatment of disorders, conditions or diseases of the ocular posterior segment.

Yet another advantage of the proposed Ceria NPs and related with the toxicity is that, according to preliminary results, the particles do not accumulate in the eye in case of overdose and any excess is quickly removed/rinsed away. This makes possible repeated administration schedules for more than one day (i.e., 7 days or more), since after seven days of application of the Ceria NPs in form of a collyrium, only a 4-fold increase of concentration was observed with indications of saturation.

Thus, in a first aspect the invention relates to a single-crystal single cerium oxide nanoparticle of formula (I):

NP-(C) (I),

wherein
NP is a single-crystal single cerium oxide (CeO₂) nanoparticle with a crystal diameter from 3 to 5 nm, and (C) is a coating of citrate molecules adsorbed on the NP,
for use in the treatment of a disorder or disease of the posterior segment of the eye, wherein the treatment comprises administration of a topical ophthalmic dose of single-crystal single cerium oxide nanoparticles of formula (I).

The nanoparticles of formula (I) are referred along this description as coated Ceria-NP, coated CeO2-NP, CeO2-NP-(C), or simply NP-(C). In any case, and if not indicated to the contrary, they relate to the nanoparticles of the single-crystal oxide comprising the citrate molecules associated to or adsorbed or coating (used as synonymous) said single-crystal.

For this topical ophthalmic administration, the nanoparticles are particularly in form of an aqueous colloidal suspension comprising the single-crystal single cerium oxide nanoparticles of formula (I) (NPs-(C)) of formula (I)), and a pharmaceutically acceptable citrate salt.

Thus, another aspect of the invention relates to aqueous colloidal suspensions comprising a pharmaceutically acceptable citrate salt and nanoparticles of formula NP-(C) (I),
wherein NP is a single-crystal single cerium oxide nanoparticle with a crystal diameter from 3 to 5 nm, and (C) is citrate adsorbed on, thus coating, the NP; being these suspensions for use in the treatment of a disorder or disease of the posterior segment of the eye, wherein the treatment comprises administration of a topical ophthalmic dose of the aqueous colloidal suspension.

These colloidal suspensions for the intended use are obtainable by preparing CeO₂ nanoparticles using a cerium (III) citrate complex as a precursor and the pH basic precipitation in the presence of an excess of the pharmaceutically acceptable citrate salt, this excess defined as an amount higher than the one needed to fully cover the CeO₂ nanoparticle available surface. Inventors have determined that for 1 mg/ml of CeO₂ nanoparticles the amount in excess of the pharmaceutically acceptable salt of citrate in the colloidal suspension is from 2 to 10 mM in order to have a stable active principle that is further dispersed, for example, in a methylcellulose solution or other cellulose polymers (see examples below where citrate coated CeO₂NPs of formula (I), were dispersed into a solution containing 5% of methylcellulose to get a CeO₂ final concentration of 2 mg/ml of CeO₂).

Thus, it is also herewith disclosed an aqueous colloidal suspension comprising a pharmaceutically acceptable citrate salt and nanoparticles of formula NP-(C) (I), obtainable by:
(i) mixing in an aqueous medium a salt of cerium, in particular cerium nitrate, with an amount of a pharmaceutically acceptable citrate salt, in particular a citrate salt of an alkali or alkaline-earth element and which amount of citrate salt is in excess in relation to the amount of the salt of cerium, and let the reaction mixture under stirring for an adequate time, while media is conducted at a basic pH, to get cerium (III) complexes with citrate be oxidized to cerium (IV) complexes before mineralization, that is, before the formation of the cerium oxide;
(ii) allowing the reaction mixture to reflux at a temperature of at least100 °C for a period of time to obtain a suspension with dispersed CeO₂ nanoparticles (NP), which are single-crystal single cerium oxide nanoparticle with a crystal diameter from 3 to 5 nm, and that comprise citrate (C) adsorbed on, thus coating, the NP.

Note that in absence of the citrate salt, such as sodium citrate (SC), Cerium nitrate will precipitate in form of cerium oxide at RT (room Temperature) and basic pH, forming aggregates. However, the stability of the cerium bond with the salt of citrate (e.g., Ce-SC bond) prevents this, and high temperature (T) for a period of time is needed then to break the complex bonds and to obtain the CeO₂ NPs (mineral) coated with the citrate salt (i.e., SC coated CeO₂ NPs).

The colloidal suspensions of Ceria NPs of formula (I) include these nanoparticles as single-crystals (i.e. no aggregation of particles but isolated) with a diameter allowing the entering to the ocular posterior segment without precipitation in the aqueous suspension, in part due to the presence of the citrate molecules adsorbed on the single-crystal, or in other words making a kind of coating around it. Citrate molecules are associated by ionic or van der Waals forces with the cerium oxide surface of the nanoparticles, which acquire a net negative charge. Thus, colloidal particles are negatively charged. Despite the low size of the particles, therapeutic amounts surprisingly entered and reached retina without the aid of nanoparticle functionalization. Thus, in the aqueous colloidal suspension the nanoparticles surface is free of any functionalization by molecules of hydrophobic or amphiphilic nature (i.e., polyethylene glycols, saccharides) and free of encapsulating carriers (i.e., liposomes, exosomes, etc.). Avoidance of functionalization is of interest, since the more complex the molecules, the more reactive generally to immune system of the host.

Considering the state of the art, it was unexpected that so simpler (without functionalization) nanoparticles of a very small size did not aggregate and did reach retina and other posterior segment parts of eye once applied topically onto the eye surface.

A third aspect of the invention is a pharmaceutical or veterinary composition comprising:
(a) single-crystal single cerium oxide nanoparticles of formula (I):

   NP-(C) (I),

   wherein NP is a single-crystal single cerium oxide nanoparticle with a crystal diameter from 3 to 5 nm, measured with transmission electron microscopy and X-Ray diffraction, and (C) is citrate molecules adsorbed on, thus coating, the NP; and
(b) a pharmaceutically or veterinary acceptable citrate salt, together with pharmaceutically or veterinary acceptable excipients and/or carriers.

These compositions, when formulated in particular as pharmaceutical or veterinary topical eye colloidal suspensions comprising the nanoparticles in colloidal suspension and the pharmaceutically or veterinary acceptable citrate salt, allow high penetration percentages of the active into the posterior segment, and a uniform distribution within all the structures therein, although topically applied (i.e., through cornea, sclera or conjunctiva).

### BRIEF DESCRIPTION OF THE DRAWINGS

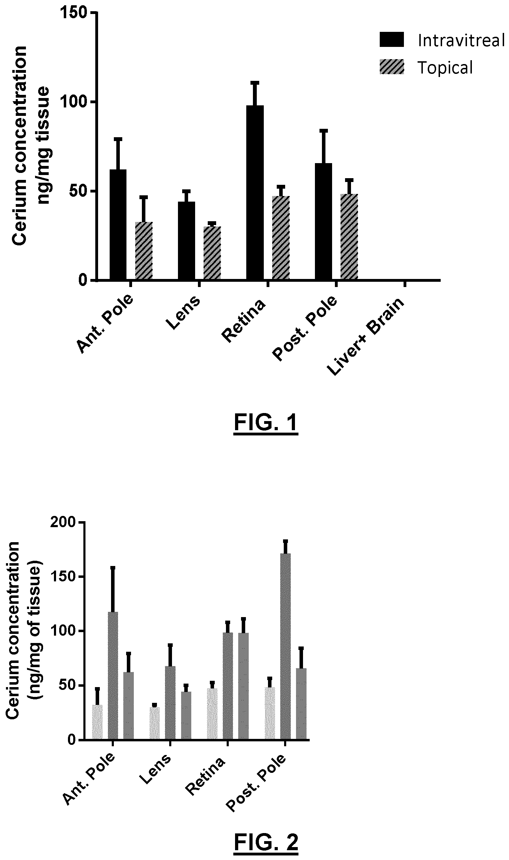

FIG. 1 is a graphic of bars with cerium concentration (ng/mg tissue), detected by ICP-MS (Y- axis) in the different parts of eye (X-axis), after intravitreal (black bars) or topical administration (pattern bars). *Ant. Pole* means anterior pole including cornea and iris. *Post. pole* means posterior pole or segment of eye including retinal pigment epithelium, sclera and choroid).
FIG. 2 is also a graphic of bars with cerium concentration (ng/mg tissue), detected by ICP-MS (Y-axis) in the different parts of eye (X-axis). Left column in each set of bars indicates amount detected 24h after topical administration (8.6 µg). Column in the middle of each set of bars indicates amount detected 7 days (7d) after a daily topical administration (60.2 µg in a week; 8.6 µg/day). Right bar of each set of bars indicates amount detected 24 h after intravitreal administration (2 µg). *Ant. Pole* means anterior pole including cornea and iris. *Post. pole* means posterior pole or segment of eye including retinal pigment epithelium, sclera and choroid).

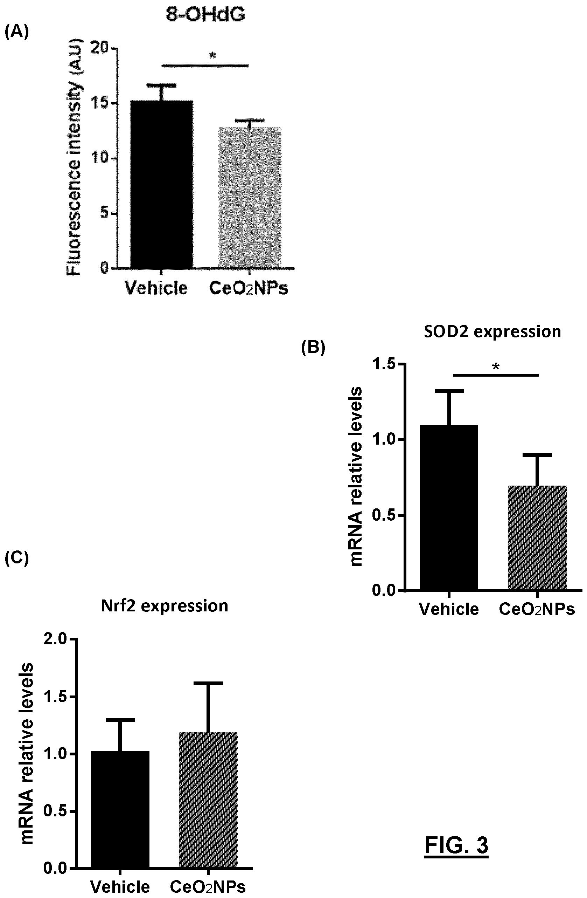

FIG. 3 is a graphic showing for 8-OHdG, fluorescence intensity in arbitrary units (a.u.) detected in an assay with vehicle (fresh cell culture media) and the aqueous colloidal suspension comprising citrate coated CeO₂NPs (i.e. NP-(C)) (FIG. 3(A)). FIG. 3(B) and FIG. 3(C) show mRNA relative levels SOD2 and Nrf2 expression, respectively.

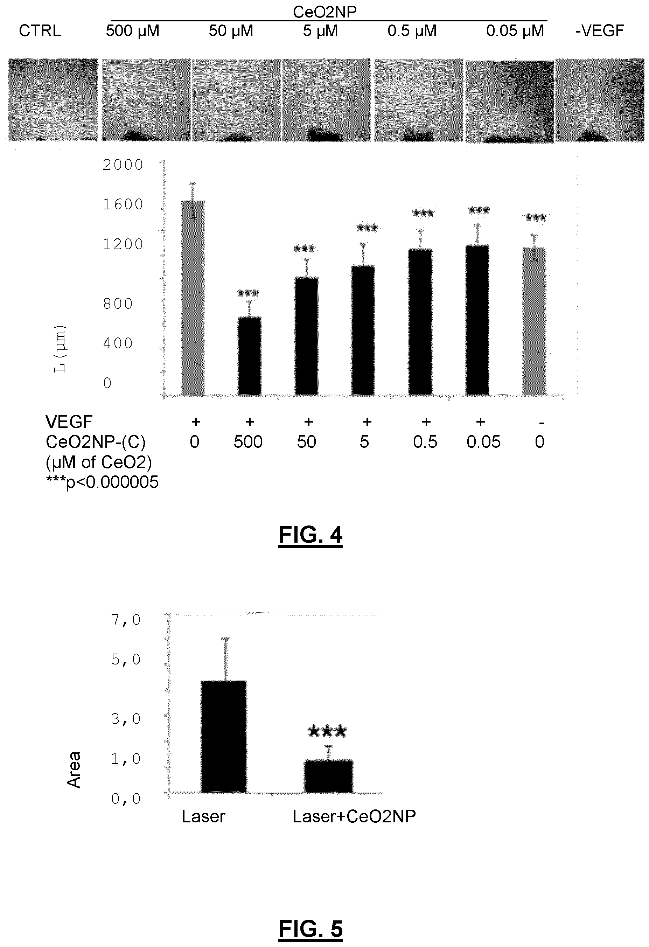

FIG. 4 illustrates angiogenesis (microvessel length (L) in micrometers) tested for different concentrations of CeO₂NP-(C) of formula (I) from 0.05 to 500 µM of CeO₂ for 7 days in a choroid sprouting assay. Medium was changed every 48 hr. Images were taken using an Olympus microscope. Microvessels length was quantified using ImageJ software. VEGF is the positive control.
FIG. 5 is a graphic that shows anti-angiogenic activity of CeO₂NP-(C) of formula (I) in an *in vivo* mouse model of choroidal neovascularization induced by laser. Area is the area of lesion.

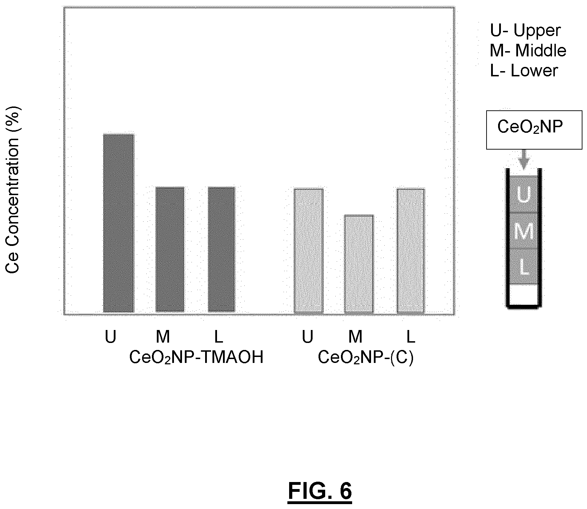

FIG. 6 shows the percentage concentration of CeO₂NP distributed along a synthetic vitreous humor. CeO₂NP-TMAOH corresponds to an aqueous suspension comprising state of the art nanoparticles prepared with tetramethylamoniun hydroxide and that do not contain citrate molecules. CeO₂NP-(C) corresponds to an aqueous suspension of ceria nanoparticles according to the invention prepared in the presence of sodium citrate. Each bar illustrates the concentration of cerium measured at the different levels (upper U, middle M or lower L) of the simulated (i.e., synthetic) vitreous humor.

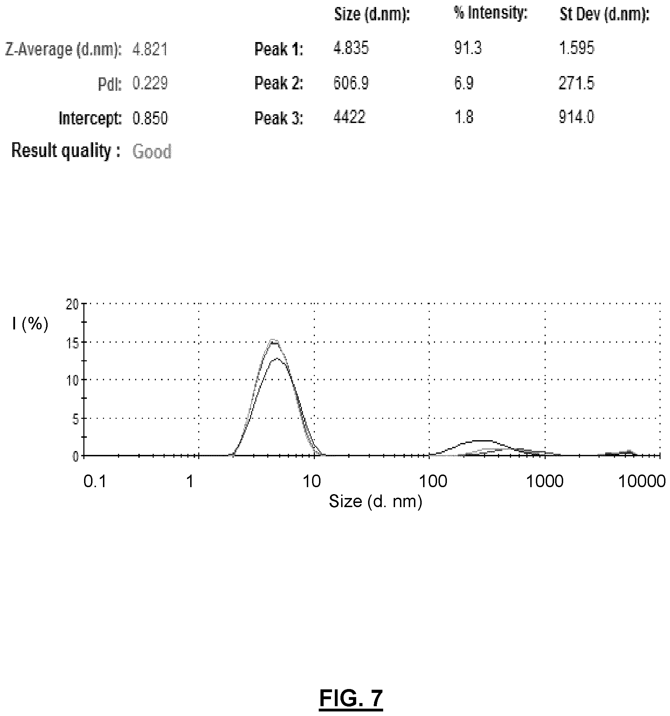

FIG.7 shows the Dynamic Light Scattering (DLS) analysis of the nanoparticles for use in the invention. It displays the peaks of the size distribution (Size in nm) by intensity (I %). DLS showed a well-defined peak by intensity around 5 nm which corresponds to single dispersed CeO₂ NPs. Other less intense peaks at 100's of nm were sometimes observed. These large peaks appeared even after filtering the sample with a 300 kDa (10 nm) cellulose filter, thus inventors consider that they are artifacts due to the low scattering of these ultrasmall nanoparticles. Further corroboration of this is the fact that after filtering the solution with a 10 nm pore filter, most of the absorbance of the particles was preserved. This peak disappears when the data is visualized by number rather than intensity. The picture illustrates three records with nanoparticles obtained with Ce 10 mM and sodium citrate 20 mM with TMAOH at 25 mM and filtered.

### DETAILED DESCRIPTION OF THE INVENTION

The invention is defined by the claims.

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

By "single-crystal single cerium oxide nanoparticle" it is to be understood that said nanoparticle is not aggregated (by weak or strong physical interaction) with other nanoparticles. Thus, it is isolated as a mono-nanoparticle or monodispersed. They are also termed "single crystallites of cerium oxide". For example, single 3-5 nm crystallites of cerium oxide. All these structures can be seen by TEM. With the TEM technology high electron density of the Cerium atoms coupled to oxygen can be visualized. With X-ray technology crystal domains (group of atoms in columns and rows in the same crystal structure) are detected. According to this description, single-crystal or monocrystal means that there is only one crystal of cerium oxide configuring the nanoparticle, which has a particular crystal diameter. Being said nanoparticles single nanoparticles and single-crystal, the X-ray diffraction detected crystal domain is highly similar with the diameter detected by TEM, which is the prove that nanoparticles are not aggregated, as DLS measurements corroborate (FIG.7).

The term "nanoparticle" as used herein, refers generally to a particle with at least two dimensions at the nanoscale, particularly with all three dimensions at the nanoscale (1-100 nm). In this description, the nanoparticles are in the range 3 nm to 5 nm, including 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9 and 5. More particularly in the range from 3.0 to 3.5 nm. As regards the shape of the nanoparticles described herein, there are included spheres and polyhedral. In a particular embodiment the nanoparticle is spherical.

As used herein, the term "size" refers to a characteristic physical dimension. For example, in the case of a nanoparticle that is substantially spherical, the size of the nanoparticle corresponds to the diameter of the nanoparticle. When referring to a set of nanoparticles as being of a particular size, it is contemplated that the set of nanoparticles can have a distribution of sizes around the specified size. Thus, as used herein, a size of a set of nanoparticles can refer to a mode of a distribution of sizes, such as a peak size of the distribution of sizes. In addition, when not perfectly spherical, the diameter is the equivalent diameter of the spherical body including the object.

The term "cerium oxide" or "ceria" refers to cerium (III) oxide (Ce³⁺) and cerium (IV) oxide (Ce⁴⁺) species that are both present when constituting the nanoparticles. Although many of the cerium atoms in cerium oxide nanoparticles are usually in the (Ce⁴⁺) oxidation state, small cerium oxide nanoparticles also contain cerium atoms in the (Ce³⁺) oxidation state. Cerium oxide nanoparticles (abbreviated also as Ceria NPs, CeO₂-NP or simply CNP) are used in a variety of applications mainly due to its high surface area and the ability of cerium oxide to cycle between (III and IV) oxidation states. In a particular embodiment, cerium oxide nanoparticles are in the (Ce⁴⁺) oxidation state.

When in this description the diameter of the nanoparticle is mentioned, it relates to the crystal size diameter (single crystal). As previously indicated, crystal size is usually measured from X-ray diffraction patterns while particle size is measured by TEM. In the case of single-crystal nanoparticles, XRD, TEM and DLS sizes coincide. Typical crystal size diameters of the nanoparticles used in the present invention range from 3 to 5 nm, when measured by TEM, more particularly the crystal size diameter of the nanoparticles is 5 nm.

The "hydrodynamic diameter" determined by dynamic light scattering (DLS) techniques based on the Stoke-Einstein equation is used herewith to refer to the single-crystal nanoparticle with adsorbed water and other molecules in the colloidal suspension. It can be measured by illuminating the particles with a laser and analyzing the intensity fluctuations in the scattered light. Dynamic light scattering measures Brownian motion and relates it to the size of the particles for which light intensity is proportional to the square of the volume of the particle. Therefore, the hydrodynamic size is always larger than the size observed by transmission electron microscopy, this later the one of the single-crystal nanoparticles. Typical hydrodynamic diameters of the single-crystal cerium oxide nanoparticles for use in present invention (highly monodispersed nanoparticles) gave a peak around 5 nm, which was much the same as TEM and XRD)
In chemistry, a colloid is a mixture in which one substance of microscopically dispersed insoluble particles is suspended throughout another substance. Sometimes the dispersed substance alone is called the colloid; the term colloidal suspension refers unambiguously to the overall mixture. Unlike a solution, whose solute and solvent constitute only one phase, a colloid has a dispersed phase (the suspended particles) and a continuous phase (the medium of suspension) that arise by phase separation. To qualify as a colloid, the mixture must be one that does not settle or would take a very long time to settle appreciably. In case of this invention, the dispersed phase are the single-crystals of single Ceria NPs (3-5 nm) with the associated citrate (C) molecules (citrate coating) on their surface, and the continuous phase where particles are suspended is a solvent, preferably a polar solvent (i.e. water, optionally buffered) comprising also the pharmaceutically acceptable citrate salts (in excess).

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The term "pharmaceutically or veterinary acceptable" as used herein pertains to compounds, materials, compositions, and/or dosage forms which are, within the scope of sound medical and veterinary judgment, suitable for use in contact with the tissues of a subject (e.g. human or any other animal) without significant toxicity, irritation, allergic response, or other problem or complication, commensurate with a reasonable benefit/risk ratio. Each carrier, excipient, etc., must also be "acceptable" in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. Suitable carriers, excipients, etc. can be found in standard pharmaceutical texts, and include, as a way of example preservatives, agglutinants, humectants, emollients, tonicity agents to adjust osmolality, chelating agents, and antioxidants.

As above exposed, the inventors propose a new therapeutically approach for disorders, conditions or diseases of the posterior segment of the eye.

According to the first aspect, single-crystal single cerium oxide nanoparticles of formula (I):

NP-(C) (I),

wherein NP is a single-crystal single cerium oxide nanoparticle with a crystal diameter from 3 to 5 nm, and (C) is a coating of citrate molecules adsorbed on the NP, are proposed for use in the treatment of a disorder or disease of the posterior segment of the eye, wherein the treatment comprises administration of a topical ophthalmic dose of aqueous colloidal suspension.

In a particular embodiment of the first aspect, the single-crystal single cerium oxide nanoparticles have a crystal diameter from 3 to 3.5 nm measured by TEM. This means that the single-crystal single cerium oxide nanoparticles have a crystal diameter selected from 3.0, 3.1, 3.2, 3.3, 3.4, 3.5 nm, as the mean of crystal diameter of the distribution of sizes.

The second aspect of the invention relates to aqueous colloidal suspensions comprising a pharmaceutically acceptable citrate salt and nanoparticles of formula NP-(C) (I),
wherein NP is a single-crystal single cerium oxide nanoparticle with a crystal diameter from 3 to 5 nm, and (C) is citrate adsorbed on, thus coating, the NP; being these suspensions for use in the treatment of a disorder or disease of the posterior segment of the eye, wherein the treatment comprises administration of a topical ophthalmic dose of aqueous colloidal suspension.

In a particular embodiment of these aqueous colloidal suspensions for use according to this second aspect, the single crystal single cerium oxide nanoparticles have a crystal diameter from 3 to 3.5 nm measured by TEM. This means that the single crystal cerium oxide nanoparticles have a crystal diameter selected from 3.0, 3.1, 3.2, 3.3, 3.4, 3.5 nm, as the mean of crystal diameter of the distribution of sizes.

In another particular embodiment of the aqueous colloidal suspensions, for use as indicated, optionally in combination with any of the embodiments above or below, the pharmaceutically acceptable citrate salt in the colloidal suspension is selected from an alkaline and an alkaline-earth metal salt, or combinations thereof. Thus, in a more particular embodiment it is selected from a pharmaceutically acceptable lithium citrate, sodium citrate, potassium citrate, beryllium citrate, magnesium citrate, calcium citrate, strontium citrate, barium citrate and radium citrate. In a more particular embodiment is sodium citrate.

Aqueous colloidal suspensions for use according to the second aspect comprise, in a particular embodiment, the pharmaceutically acceptable citrate salt at a final concentration from 1.0 to 20 mM. More in particular, from 2.0 to 10 mM.

Inventors tested several concentrations of the nanoparticles of formula (I) in the aqueous colloidal suspension in an *in vivo* model. Doses of the topical ophthalmic route could be adjusted from 1.0 to 250 micrograms of the CeO₂ in the form of the nanoparticles of formula (I) per day (micrograms/eye/day). These doses include very low, but effective doses. This supposes the advantage of null side-effects while effectivity is maintained, precisely due to the "catalytic behavior" of ceria. Moreover, final pharmaceutical composition can be afforded for an appropriate cost to consumer.

Doses and concentrations of the nanoparticles of formula (I) in this description are indicated using the administered amount in micrograms/eye/day of cerium oxide (which is in the form of the citrated coated nanoparticles), or the concentration of this cerium oxide in the administered composition (in mg/ml or in the equivalent molarity according to cerium oxide molecular weight; 172.11 g/ml). For the quantification of cerium oxide in any of its oxidation states there are spectrophotometric and mass spectroscopy techniques the skilled person in the art will know.

The aqueous colloidal suspension for use according to this aspect of the invention is, in a particular embodiment, an eye drop colloidal suspension. This means that the colloidal suspension comprising the ceria NPs of formula (I) and the pharmaceutically acceptable citrate salt, also includes excipients and carriers, such as buffer systems and preservatives. The pharmaceutical or veterinary topical eye drop colloidal suspension are to be understood as topical eye compositions applicable to the cornea, sclera or to the conjunctival fornix.

As indicated, particular ophthalmic doses of cerium oxide administered in this form of nanoparticles of formula (I) in the aqueous suspensions are from 1.0 to 250.0 micrograms of cerium oxide/day (i.e., micrograms/eye/day). In a more particular embodiment, the ophthalmic doses are from 1.0 to 100.0 micrograms/day. In another more particular embodiment, they are from 2.0 to 50.0 micrograms/day, even more in particular from 5.0 to 50.0 micrograms/day. When these aqueous colloidal suspensions are in form of eye drops they are generally used in volumes of 30 to 50 microliters/drop, more in particular 50 microliters/drop. These doses can be applied once a day or more than once a day. They can also be maintained for one or several weeks.

More in particular the ophthalmic dose of cerium oxide is administered from an aqueous colloidal suspension comprising from 1.0 to 5.0 mg/ml, more in particular from 2.0 to 4.0 mg/ml of cerium oxide as the single-crystal single cerium oxide nanoparticles of formula (I), stabilized in the excess of the pharmaceutical citrate salt. Thus, in the aqueous colloidal suspension comprising the single-crystal single cerium oxide nanoparticles of formula (I), cerium oxide is at a concentration selected from 1.0, 2.0, 3.0, 4.0, 5.0 mg/ml. These concentrations allow the administering of ophthalmic doses of cerium oxide in the nanoparticle form that range from 50 µg to 250 µg per drop of 50 µl, for example.

In a more particular embodiment of the aqueous suspension for the proposed use, it comprises 2 mg/ml of the cerium oxide in form of the single-crystal single cerium oxide nanoparticles of formula (I), wherein the crystal diameter of CeO₂NPs is of 3.5 nm measured by TEM, and wherein the final ophthalmic dose of the cerium oxide in form of the single-crystal single cerium oxide nanoparticles of formula (I) is from 1.0 to 250 micrograms/eye/day.

Many are the diseases of the posterior segment of eye. The aim of this invention is to focus on every ocular disease of the posterior segment, in which there is an inconvenient pro-inflammatory state and general oxidative stress, mainly caused by ROS, which damage the structures and worsens the disease, if already manifested, from an acute stage to a chronic stage.

Thus, in a particular embodiment of the single-crystal single nanoparticle or the aqueous colloidal suspension comprising it, for use as indicated, the eye disease is any disorder of any of the structures or tissues of the posterior segment, in that beside the primary disease cause (genetic, multifactorial, trauma, cancer, ...), inconvenient pro-inflammatory state and general oxidative stress environment is present. This is common to all diseases listed below.

Thus, in a particular embodiment of the first and second aspects, the single-crystal single nanoparticle and the aqueous colloidal suspension is for use in the prevention and/or treatment of a condition or disease of the posterior segment of the eye selected from the group consisting of retinal and/or choroid pathologies, vitreous humour pathologies, posterior sclera pathology, optic nerve pathologies, intraocular tumors, and combinations thereof.

In a more particular embodiment, optionally in combination with any embodiment above or below of the single-crystal single nanoparticle or of the colloidal suspension for use of the first and second aspects, the disease of the posterior segment of the eye is a retinal pathology and/or choroid pathology selected from a retinal vasculopathy, a maculopathy, an hereditary eye fundus dystrophy, an idiopathic chorioretinopathy, a central serous retinopathy, generalized choroidal dystrophy, retinoblastoma, and combinations thereof.

In a more particular embodiment, the single-crystal single nanoparticle or the aqueous colloidal suspension comprising the nanoparticles is for use in the prevention and/or treatment of a retinal vasculopathy selected from diabetic retinopathy, diabetic papillopathy, non-diabetic retinopathy, ocular ischemic syndrome, hypertensive retinopathy, thalassemia retinopathy, Coats' syndrome, Eales' syndrome, radiation retinopathy, solar retinopathy, purtscher retinopathy, polypoidal choroidal vasculopathy (PCV), retinal macroaneurysm, retinal microaneurysm, leukemic retinopathy, retinal ischemia, chronic retina disorders, and combinations thereof. In a more particular embodiment, the single-crystal single nanoparticle or the aqueous colloidal suspension is for use in the prevention and/or treatment of diabetic retinopathy, even more in particular early stages of the disease when intravitreal injections are not adequate due to their associated risks.

Indeed, as previously indicated, current aqueous colloidal suspensions for the intended use with the nanoparticles of formula (I), or this nanoparticles as such, are real substitutes of intravitreal injections, making them in a particular embodiment for use in early stages of the conditions or diseases listed in this description without any risk for the subject. This is of particular interest for the known as dry form of aged-related macular degeneration (AMD) and for diabetic retinopathy.

Thus, in another particular embodiment of the first aspect, the single-crystal single nanoparticle, or the aqueous colloidal suspension comprising it is for use in the prevention and/or treatment of a maculopathy selected from aged-related macular degeneration (AMD), retinal angiomatous proliferation, polipoidal choroidal vasculopathy, malattia leventinese, full thickness macular hole, macular epiretinal membrane, macular telangiectasias, cellophane maculopathy or macular pucker, myopia maculopathy, exudative maculopathy after venous thrombosis of the retina, acute macular neuroretinopathy, macular cystoids, macular edema, retinal angioid streaks, choroidal folds, hypotony maculopathy and combinations thereof; and wherein the hereditary eye fundus distrophy is selected from the group consisting of retinitis pigmentosa; atypical retinitis pigmentosa including Usher's syndrome, retinitis punctata albicans, Leber's congenital amaurosis, dystrophy of the cones, rod dystrophy, Bietti crystalline corneoretinal dystrophy, juvenile macular dystrophy, all types of macular dystrophy, Stargardt's disease or Fundus flavimaculatus, Usher's Syndrome and combinatios thereof.

In another more particular embodiment, the single-crystal single cerium oxide nanoparticle of formula (I), or the aqueous colloidal suspension, as above disclosed, is for use in the prevention and/or treatment of AMD selected from wet AMD and dry AMD (geographic atrophy). In a more particular embodiment is for use in dry AMD. In another particular embodiment is for use in the prevention and/or treatment of retinitis pigmentosa.

In another particular embodiment, the single-crystal single cerium oxide nanoparticle of formula (I), or the aqueous colloidal suspension comprising it, is for use in the prevention and/or treatment of a vitreous humour pathology selected from the group consisting of sub-macular and vitreous haemorrhages, asteroid hyalosis, vitreous detachment, eye floaters or myodesopsia, hereditary vitreoretinopathies, Stickler's syndrome or Wagner's Syndrome, and combinations thereof.

In also another particular embodiment, the single-crystal single cerium oxide nanoparticle of formula (I), or the aqueous colloidal suspension comprising it, is for use in the prevention and/or treatment of an optic nerve pathology selected from the group consisting of optic atrophy, optic neuritis, neuroretinitis, ischemic neuropathy, hereditary optic neuropathy, toxic amblyopia or nutritional optic neuropathy, ocular hypertension, primary glaucoma , secondary glaucoma, iridocorneal endothelial syndrome associated to glaucoma, head drusen or optic disc drusen, papilledema and combinations thereof. In a more particular embodiment is for use in the prevention and/or treatment of primary glaucoma or secondary glaucoma.

Inventors have developed particular eye colloidal suspensions to put the proposed use into practice.

Thus, as previously indicated, another aspect of the invention relates to a pharmaceutical or veterinary composition comprising:
(a) a therapeutically effective amount of single-crystal single cerium oxide nanoparticles of formula (I):

   NP-(C) (I)

   , wherein NP is a single-crystal single cerium oxide nanoparticle with a crystal diameter from 3 to 5 nm, measured with transmission electron microscopy and X-Ray diffraction, and (C) is citrate (i.e., citrate molecules) adsorbed on, thus coating, the NP; and
(b) a pharmaceutically or veterinary acceptable citrate salt, together with pharmaceutically or veterinary acceptable excipients and/or carriers.

In a particular embodiment of the pharmaceutical or veterinary composition, it is a pharmaceutical or veterinary topical eye colloidal suspension comprising:
(a) a therapeutically effective amount of single-crystal single cerium oxide nanoparticles of formula (I):

   NP-(C) (I),

   wherein NP is a single-crystal single cerium oxide nanoparticle with a crystal diameter from 3 to 5 nm, measured with transmission electron microscopy and X-Ray diffraction, and (C) is citrate adsorbed on, thus coating, the NP; and (b) a pharmaceutically or veterinary acceptable citrate salt, together with topical ophthalmic pharmaceutically or veterinary acceptable excipients and/or carriers.

In a more particular embodiment of the pharmaceutical or veterinary topical eye colloidal suspension, it comprises single-crystal single cerium oxide nanoparticle of formula (I) giving an amount in the suspension from 1.0 mg/ml to 5.0 mg/ml of the cerium oxide. More in particular, it comprises single-crystal single cerium oxide nanoparticle of formula (I) giving an amount from 2.0 mg/ml to 4.0 mg/ml of cerium oxide; even more in particular of 2.0 mg/ml).

In another particular embodiment, the pharmaceutically acceptable citrate salt in the pharmaceutical or veterinary composition, being in particular a pharmaceutical or veterinary topical eye colloidal suspension, is at a final concentration from 1.0 to 20 mM. More in particular, from 2.0 to 10 mM.

In also another particular embodiment of the topical eye aqueous colloidal suspension, optionally in combination with any of the embodiments above or below of these topical eye aqueous colloidal suspensions, it has a viscosity from 3000-5500 mPa.

When in this description it is indicated that a composition has a particular viscosity within a range, it is related to the dynamic viscosity. Thus, the pharmaceutical compositions of the invention have a dynamic viscosity from 3000 to 5500mPa, at room temperature (i.e., 20 ±0.1 °C) and normal atmospheric pressure. According to the European pharmacopoeia (8th edition, 2.2.8 "Viscosity"), dynamic viscosity or viscosity coefficient η is the tangential force per unit surface, known as shearing stress T and expressed in pascals, necessary to move, parallel to the sliding plane, a layer of liquid of 1 square meter at a rate (v) of 1 meter per second relative to a parallel layer at a distance (x) of 1 meter. The ratio dv/dx is a speed gradient giving the rate of shear D expressed in reciprocal seconds (s⁻¹), so that η=T/D. The unit of dynamic viscosity is the pascal second (Pa.s ).

In also another particular embodiment of the topical eye aqueous colloidal suspension, optionally in combination with any of the embodiments above or below of these topical eye aqueous colloidal suspensions, it comprises single-crystal single cerium oxide nanoparticles of formula (I) (i.e. citrate coated CeO₂NPs) dispersed in a polar solvent, said solvent comprising water, a citrate salt (i.e. sodium citrate), the viscosity agent hydroxypropyl methylcellulose, the preservatives benzalkonium chloride and boric acid, and the tensoactive amino hydroxy methyl propanediol.

Additionally, the aqueous (i.e., for eye drop) colloidal suspensions of the present invention may contain other ingredients, such as fragrances, colorants, and other components known in the state of the art for use in topical eye formulations.

Topical eye compositions of the present invention can be prepared according to methods well known in the state of the art. The appropriate excipients and/or carriers, as well as any pH buffer, and their amounts, can readily be determined by those skilled in the art according to the type of formulation being prepared.

Components in the topical eye (ophthalmic) composition of the invention include, in some particular embodiments, tensoactives, solvents (organic and inorganic solvents; i.e. water), viscosity agents, preservatives, agglutinants, emollients, and antioxidants, isotonifying and/or isoosmozing agents, and mucoadhesive polymers.

Other viscosity agents are in particular polyvynil alcohol, compounds derived from cellulose such as methylcellulose and hydroxypropylmethylcellulose, carbomers, PEG and mixtures thereof. Other preservatives are in particular, benzoic acid, p-hydroxybenzoic esters of C1-C4-alkyl chains (i.e. methyl-, ethyl-, propyl-, and butyl-p-benzoate), chlorobuthanol, benzyl alcohol, and mixtures thereof.

Isotonifying and/or isoosmozing agents, are in particular sodium chloride, dextrose, trehalose, mannitol, amino acids and mixtures thereof.

Excipients used as pH buffers are those allowing a pH from 4.5 to 9.0, more in particular from 4.5 to 8.5, even more in particular from 6.0 to 8.2, and preferably from 7.0 to 8.1, even more preferably from 7.5 to 8.0. Examples of pH buffers include citrate salts (citric acid/citrate buffer), which is this case is constituting the aqueous colloidal suspension, phosphate salts (phosporic acid/phosphate buffer), borate salts (boric acid/borate buffer), and mixtures thereof, all salts being those pharmaceuticaly accepatble. pH buffers may in additional comprise amino acids, in particular arginine, lysine, and an amine-derived compound selected from methylglucamine and trometamol, and mixtures thereof.

The pharmaceutical or veterinary composition comprising: (a) a therapeutically effective amount of single-crystal single cerium oxide nanoparticles of formula (I); and (b) a pharmaceutically or veterinary acceptable citrate salt, together with pharmaceutically or veterinary acceptable excipients and/or carriers, are in a particular embodiment in a form selected from solutions, creams, lotions, unguents, emulsions, and suspensions. These particular compositions comprise, in even a more particular embodiment, topical pharmaceutically or veterinary acceptable excipients and/or carriers. More in particular, these topical compositions, in any of the forms, are also conceived for application onto skin and mucosae.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention, which is defined by the claims.

### EXAMPLES

### EXAMPLE 1. Synthesis and characterization of cerium oxide nanoparticles and eye-drop colloidal suspension.

### MATERIALS AND METHODS:

### Synthesis of CeO₂NP-(C) (also termed NP-(C)) of formula (I):

A 50 mL solution of tetramethylamoniun hydroxide (TMAOH) 50 mM was added to a pre-formed 50 mL of a solution containing 20 mM of CeNO₃ and 40 mM of sodium citrate (SC) in a 250 mL glass bottle where the Ce-SC complex is spontaneously formed. The reaction mixture was then left under stirring at room temperature overnight. During this time the colour of the solution changed from colourless to brown and finally yellow, which indicated the oxidation of Ce³⁺ to Ce⁴⁺ and the formation of Ce⁴⁺-citrate complexes. Later, the reaction mixture was added to a three necked round bottom flask and left under refluxing at 100°C for 4h to break Ce-SC complexes and allow Ce oxide mineralization. During this time, the colour of the solution changed from yellow to pale yellow. This pale-yellow clear solution is in accordance with the typical appearance of well dispersed CeO₂NPs. Precipitation was not observed. Thus, an aqueous colloidal suspension comprising CeO₂NPs and citrate adsorbed on (or coating) this CeO₂NPs was obtained. The nanoparticles were single-crystal single cerium oxide nanoparticles of formula (I) (NP-(C)), as revealed by TEM (see below in Results section), with CeO₂NPs colloids of 3.0-3.5 nm diameter stable in water comprising the citrate salt, being part of the citrate associated or adsorbed (i.e. coating) on the single-crystal.

### Eye drop formulation:

A solution of Methocel^{®} 20% (Omnivision AG, Neuhausen, Switzerland) was used to prepare the CeO₂NPs-based eye drops. 1 ml Methocel^{®} contains, according to the manufacturer specifications, 20 mg hydroxypropyl methylcellulose, 0.1 mg benzalkonium chloride, boric acid, amino hydroxy methyl propanediol and water. It has a viscosity of 3000-5500 mPa.s. To prepare the CeO₂NPs-based eye drops, the Methocel^{®} 20% was diluted four times in distilled H₂O (thus, hydroxypropyl methylcellulose was at 5% of weight), and citrate coated CeO₂NPs of formula (I) were dispersed into this solution at a final concentration of 2 mg/ml of CeO₂ under vigorous stirring. In these conditions, the prepared eye drop formulation was able for pipetting and thus, no errors in the administered dose could be induced by the solution viscosity.

Similar eye drop formulations could also be prepared with hyaluronic acid.

### Cell culture:

ARPE19 cells (commercially available) were maintained in Dulbecco's modified Eagle's medium/nutrient mixture F-12 (DMEM/F12), supplemented with 10% Fetal Bovine Serum (FBS) and 1% penicillin-streptomycin, in a humidified incubator at 37°C and 5%CO₂. For all experiments, cells between passages 6 and 12 were used.

### Viability assay:

ARPE19 cells (commercially available from ATCC) were seeded in 24-well plates (Sarstedt) at a density of 100.000 cells/ml in complete medium (DMEMF12 + 10% FBS + antibiotic) and incubated for 36h. Cerium oxide nanoparticles of formula (I) were added at final concentrations of cerium oxide of 10 nM, 50 nM, 100 nM and 500 nM (0.0017, 0.0086, 0.0172 and 0.0860 µg/ml) in fresh media and cells were incubated for 24 or 48h more. 100µl of MTT (3-(4,5-Dimethylthiazol-2-yl)-2,5-Diphenyltetrazolium Bromide) were added to each well and cells were incubated 4h. After that, the media was removed and 300µl of DMSO were added to solubilize the formazan crystals. The optical density of each well was measured at 570nm using a microplate spectrophotometer. Data was normalized to untreated cells.

### Reactive oxygen species (ROS) levels measurements:

Intracellular ROS levels were quantified using the H₂DCFDA probe. In brief, ARPE19 cells were seeded in 24-well plates at a density of 100.000 cells/ml in complete medium and incubated for 36h. Cerium oxide nanoparticles of formula (I) were added at final concentrations of cerium oxide of 1 nM, 5 nM, 10 nM, 20 nM, 50 nM, 100 nM and 500 nM (from 0.00017 µg/ml to 0.086 µg/ml) in fresh media and incubated for 24h. Cells were washed with PBS 1X , H₂DCFDA was added in fresh medium at a final concentration of 10µM and incubated for 30min. Cells were washed again with PBS 1X and exposed to H₂O₂ (hydrogen peroxide), Antimycin A or tBH (tert-Butyl hydroperoxide) for 1h. Cells were then incubated with lysis buffer for 5min at RT, transferred into a 96-well black plate (VWR) and read immediately in a spectrofluorimeter (Excitation wave length. 485/20; Emission wavelength 528/20). ROS levels were expressed as ratio of mean intensity of sample/mean intensity of control cells.

### Animals:

Male 8-weeks old C57BL/6N mice purchased at Charles River (France) were used in CeO₂NPs of formula (I) biodistribution experiments. Mice were maintained at CELLEX facilities under standard cyclic light conditions (12h light/12h darkness), free access to filtered water and *ad libitum* food. All experiments were performed according to the Association for Research in Vision and Ophthalmology statement for the use of animals in vision and ophthalmic research and approved by the Vall d'Hebron Research Institute Ethical Committee for Animal Research (VHIR-CEEA) and local governmental authorities.

### Intravitreal and topical administration:

For intravitreal administration, mice were anesthetized with inhaled 2% isofluorane (Aerrane^{®}, Baxter laboratories) in an induction chamber and their pupils were dilated with one drop of Tropicamide (Alcon Laboratories). Each animal was afterwards placed on a heating pad with an anaesthetic mask. Under a surgical microscope, a 36-gauge bevelled needle (Hamilton) was inserted in the sclera at approximately 1mm from the superior limbus and 1µl of the correspondent solution (2 mg/ml) was administered (2 µg CeO₂ total). The eye fundus was visualized right after in a Micron III (Phoenix Research Lab) platform to corroborate the correct injection.

For the topical administration, mice were slightly anesthetized with inhaled 2% isofluorane to guarantee a correct absorption and 5µl (10 µg CeO2 total) of the correspondent treatment (eye drop formulation of CeO₂NPs of formula (I) giving 2 mg/ml of cerium oxide in the formulation) where placed on the cornea surface of each eye with a micropipette. Mice received the same treatment in both eyes to avoid cross-contamination.

### Inductively coupled plasma mass spectrometry (ICP-MS):

Mice were euthanized with inhaled CO₂ at designated time points post injection. Eyes were enucleated and fixed in 4% PFA (paraformaldehyde - dissolved in 1X PBS) for 30min before dissection under a stereomicroscope. Different ocular parts including anterior pole (cornea and iris), lens, retina, posterior pole (retinal pigment epithelium, choroid and sclera) and optic nerve were dissected and stored at -80°C until digestion. The brain and liver of each animal were also removed, immediately dry frozen and stored at 80°C. Organs were digested in concentrated HNO3 in a 1900 W microwave digestion oven (Milestone, ETHOS EASY). The resultant digestions were diluted with HNO3 1% (v/v) before being injected in the ICP-MS instrument (Agilent, 7500ce).

### Histology:

At the designated time points post-injection, mice were anesthetized with 1ml ketamine/0.3ml xylazine and transcardially perfused with 4%PFA. Eyes were enucleated and fixed with 4% PFA for 4h at RT and embedded in paraffin blocks. Eye sections of 3µm were cut in a microtome and stored at 4°C. For histological evaluation, slides were deparaffinised in xylene and rehydrated in ethanol at decreasing concentrations. The sections were fixed in ice-cold methanol/acetic acid solution (95:5 v/v) for 1min. Haematoxylin and eosin staining was carried out with a standard protocol and slides were mounted with DPX mounting medium (VWR) and visualized in the microscope.

### Immunofluorescence / TUNEL:

Slides were deparaffinised, rehydrated and fixed as described above. After the fixation, slides were washed three times with PBS 1X and antigen retrieval was performed by immersing them in sodium citrate buffer pH 6 and heating them in a pressure cooker for 4min. Samples were blocked with a solution containing 1% (v/v) BSA, 3% (v/v) NGS and 0.5% (v/v) Tween-20 in PBS for 1h at room temperature. Primary antibodies were incubated overnight at 4°C.

The following day, samples were washed with PBS and secondary antibodies were incubated 1h at room temperature in darkness. Samples were mounted with Fluoroshield with DAPI and visualized in the confocal microscope (FluoView ASW 1.4; Olympus). Primary antibodies used were rabbit anti-GFAP (ab7260, Abcam) and mouse anti-8-OHdG (ab62623, Abcam). Secondary antibodies were Alexa Fluor anti-rabbit 488 (A11008, Thermo) and Alexa Fluor anti-mouse (A11004, Thermo). For the TUNEL staining, slides were deparaffinised as explained above and stained with the TUNEL kit, following manufacturer's instructions (Click-It Plus TUNEL Assay, Invitrogen, Thermo).

### Gene expression (RNA extraction, RT and qPCR):

ARPE-19 cells were seeded in a 12-well plate at a density of 100.000cells/ml. After 36h, cerium oxide nanoparticles were added at final concentrations of 50 and 500nM in fresh media and cells were incubated overnight. Cells were exposed to tBH for 4h to induce changes in gene expression. Then, they were washed with PBS 1X and Tri Reagent (Sigma) was added into each well before scrapping the cells. RNA was extracted using manufacturer's protocol. In brief, one microgram of RNA, pre-treated with DNase I (Life Technologies, Thermo), was used to synthetize first strand cDNA using the High-Capacity Reverse Transcription kit (Applied Biosystems, Thermo) with oligo(dT) Primers (Thermo). Quantitative real-time PCR was performed with LightCycler 480 SYBR Green I Master (Roche). Genes analysed were SOD2 and Nrf2. Gene expression was normalized to GAPDH. Animals were euthanized by CO₂ inhalation and eyes were immediately enucleated. Eyes were dissected under a stereomicroscope and neural retina, EPR, choroid and sclera were frozen in liquid nitrogen. RNA from the homogenized tissue was extracted using the Tri Reagent standard protocol. Synthesis of cDNA and qPCR were performed as explained above. Genes analysed were GFAP, SOD2 and Nrf2.Gene expression was normalized to B2M.

### Choroid sprouting assay (ex vivo model of microvascular angiogenesis):

Murine eyes of C57BL/6N were immediately enucleated and kept in ice-cold medium before dissection. After removing the cornea and the lens from the anterior of the eye, the central or peripheral choroid-scleral complex was separated from the retina and cut into approximately ~2 mm×1 mm pieces (rats) or 1 mm×1 mm (mice). Choroid+sclera (here on referred to as "choroid") fragments were isolated without retinal pigment epithelium (RPE) removal by peeling RPE away with forceps and placed in growth factor-reduced Matrigel^{™} (BD Biosciences, Cat. 354230) seeded in 24 well plates. 30 µL Matrigel ^{®} was used to coat the bottom of 24 well plates without touching the edge of the well. The thickness of Matrigel ^{®}was approximately 0.4 mm. After seeding the choroid, plates were incubated in a 37°C cell culture incubator without medium for 10 minutes in order for the Matrigel^{™} to solidify. Then, 500 µL of medium EBM-2/EGM (Lonza) was then added to each well and incubated at 37°C with 5% CO2 for 48 hr before any treatment. Then, cells were treated with different concentrations of CeO2 NP-(C) of formula (I) from 0.05 to 500 µM of cerium oxide for 7 days. Medium was changed every 48 hr. Images were taken using an Olympus microscope. Microvessels length was quantified using ImageJ software.

### Laser-induced choroidal neovascularization (LI-CNV) mouse model:

The laser-induced choroidal neovascularization (LI-CNV) mouse model has been a crucial mainstay model for neovascular AMD. To develop a mouse model with reproducible LI-CNV features, retinas of mice at post-natal week 6-8 (under sedation), were exposed to an intensity of the 430 nm argon laser (4 spots, 100 ms at 250 mW, 50µm area). Then, one group was treated with CeO2NPs of formula (I)-containing eye drops (5µl/drop at 2 mg/ml of cerium oxide in form of CeO2NPs of formula (I)) and the other group was treated with vehicle, during 7 days. Lesions' progression in the exudative area was monitored using optical coherence tomography, fundus imaging and fluorescence angiography and compared with non-treated group in vivo. Eyes were enucleated and posterior pole isolated in ice-cold PBS. Choroid, sclera and RPE layers were isolated from retinas and an immunostaining was performed in flat mount conformation using Iba-1 (Dako) and Isolectin-B4. Lesions areas were quantified using ImageJ software.

### RESULTS

### Characterization of cerium oxide nanoparticles by TEM:

Ceria-NPs of formula (I) were prepared as indicated above in Materials and Methods. TEM image revealed that Ceria-NPs colloids of 3.0-3.5 nm stable in water at concentrations of 1 mg/ml were yielded.

### Cerium oxide nanoparticles are not toxic and reduce intracellular ROS levels in ARPE19 cells:

After the synthesis and characterization of CeO₂NPs-(C) of formula (I), its safety in ARPE19 cells with an MTT assay was evaluated. CeO₂NPs-(C) of formula (I) incubated during 24 and 48h did not show a reduction in cell viability, at any of the tested concentrations. To test the ability of CeO₂NPs-(C) of formula (I) to scavenge intracellular ROS in ARPE19 cells, cells were pre-treated with the nanoparticles during 24h, removed the supernatant and adding new media, before exposing them to different oxidant agents for 1h. Specifically, H₂O₂, tBH and Antimycin A (AA) were used to induce an oxidative stress response in ARPE19 cells through different pathways. In all cases, a dose-dependent decrease of the ROS levels in cells treated with CeO₂NPs-(C) of formula (I) was observed (data not shown). Optical microscope images of ganglion cell layer (GCL) slides in the case of exposure to tBH showed the same results with a decrease of fluorescent signal (8-OHdG, fluorescence intensity in arbitrary units (a.u.) (FIG. 3 (A)), indicating the presence of free radicals and their decrease as the concentration of CeO₂NPs-(C) of formula (I) increases. Once the decrease of ROS was observed, the question was if this would modify the expression of oxidative stress-related genes. Thus, the antioxidant capacity of CeO₂NPs-(C) of formula (I) in these conditions was also evaluated with quantitative real-time PCR. ARPE19 cells were pre-treated with CeO₂NPs-(C) of formula (I) during 24h before inducing oxidative stress with tBH for 4h. While the treatment with CeO₂NPs-(C) of formula (I) alone did not produce changes in superoxide dismutase-2 (SOD2) expression, cells treated with CeO₂NPs-(C) of formula (I) and with induction of oxidative stress experienced a higher increase of SOD2 expression than cells incubated just with tBH (FIG. 3(B)). CeO₂NPs-(C) of formula (I) treatment also induced the upregulation of expression of transcription factor Nrf2 expression (FIG. 3(C)). Nrf2 is known as the main antioxidant transcription factor, able to protect cells from oxidative stress by inducing the expression of ROS-neutralizing enzymes (Retinal degenerative diseases, book).

### In vivo administration. Cerium is detected in mice retinas 24h after topical administration:

Before exploring beneficial therapeutic effects of CeO₂NPs-(C) of formula (I), two question where addressed. Does the CeO₂NPs-(C) of formula (I) reach the region of interest after drop casting and does the nanoparticles translocate inside the host. For that, intravitreous vs topical administration were prepared and the Ce contents analysed by Inductively coupled plasma mass spectrometry (ICP-MS) as a function of time and organelle.

Biodistribution assays after intravitreal injection were done, and cerium was detected in the retina several weeks after. Cerium was also able to reach the retina (and all posterior segment structures) after a topical administration in a mouse model of dry AMD.

The potential of cerium oxide nanoparticles as a therapy for retina degeneration has been tested *in vivo* in the *tubby* and vldlr^{-/-} mice models and in the light-damaged rat model but in all cases, through an intravitreal administration. Given the associated complications this route of administration presents, a topical administration rises as a better option.

To elucidate whether cerium could reach the retina and posterior pole of the eye after a topical administration, a biodistribution assay was performed in C57BL/6N mice receiving CeO₂NPs-(C) of formula (I) at three different cerium oxide concentrations (0.1mM; 1mM and 10mM (0.017 mg/ml, 0.172 mg/mg and 1,72 mg/ml) via intravitreal or topical administration. Mice were euthanized 24h after administration and eyes were enucleated and dissected to quantify the amount of cerium present in the different parts of the eye.

56.8% of all cerium administered was detected in the eyes of mice receiving intravitreal administration versus an 8.2% in those receiving cerium topically. With topical administration, cerium was homogenously distributed in the different parts of the eye, detecting a 2.1% in the retina. In mice receiving intravitreal administration, cerium was preferentially retained in the retina (21.6%), although it was present in all the eye components. The anterior pole retained a 14% of all cerium administered in the vitreous cavity. Indeed, the distribution is more homogeneous by eye-drops, avoiding damaging the eye with the puncture. In principle the humorous vitreous is a gel highly hydrophilic with 10 nm porosity and negatively charged. Therefore, highly soluble (light and charged), small size and strictly no aggregation is needed for the topical ophthalmological application of the nanoparticles.

Next Table 1 shows the results of the distribution assay from a concentration of colloid CeO₂NPs-(C) of formula (I) at 10 mM (2 mg/ml of cerium oxide) in the aqueous suspension with citrate:

**Table 1: Distribution of CeO₂NPs-(C) of formula (I) (percentage %), detected by ICP-MS in different parts of eye 24 h after intravitreal or topical administration.**

| | **Administration** | **Ant. pole** | **Lens** | **Retina** | **Post. pole** | **Total** |
|---|---|---|---|---|---|---|
| 24h | IV injection (2µg) | 14% | 9,8% | 21,6% | 11,4% | 56,8% |
| | Topical (8,6µg) | 1,7% | 2,3% | 2,1% | 2,1% | 8,2% |

Despite the differences in the total amount of cerium detected with the two routes of administration, the concentration reached in the different parts with topical administration was about half of the reached with intravitreal injection. Data are provided in FIG. 1, where cerium concentration (ng/mg tissue), detected by ICP-MS is depicted for the different parts of eye after intravitreal or topical administration. No amounts were detected in liver and brain, and no systemic toxicity was observed for the tested compositions in the two modes of administration.

### Cerium is not excessively accumulated after a one-week daily topical administration:

The concentration of cerium reaching the retina after a topical administration is lower than with an intravitreal injection. Therefore, a topical delivery will need a sustained administration to reach the same concentration of cerium in the retina. CeO₂NPs-(C) of formula (I) was daily administered at a concentration of cerium oxide of 10mM for one week. After a week, the cerium concentration in the retina was the same achieved with a single intravitreal injection. These data are depicted in bar diagram of FIG. 2. In FIG. 2 cerium concentration (ng/mg tissue), detected by ICP-MS is depicted for the different parts of eye. Left column in each set of bars indicates amount detected 24h after topical administration (8.6 µg). Column in the middle of each set of bars indicates amount detected 7 days (7d) after a daily topical administration (60.6 µg after the 7 days). Right bar of each set of bars indicates amount detected 24 h after intravitreal administration (2 µg). *Ant. Pole* means anterior pole including cornea and iris. *Post. pole* means posterior pole or segment of eye including retinal pigment epithelium, sclera and choroid).

Cerium accumulated in all parts of the eye at a higher concentration that with intravitreal injection (where the sample remained at the injection point probably due to the inflammation consequence of the puncture), preferentially in the posterior pole. Overall, the amount of cerium detected after a one-week administration was much lower than all cerium administered (6.48 ±1.5 % of total cerium administered; n=4), indicating a high clearance rate.

Therefore, it can be concluded that aqueous colloidal suspensions of nanoparticles of formula (I) topically administered on the surface of eye (i.e. cornea, conjunctiva, etc.) could surprisingly reach the different structures of the posterior segment in an effective mode. Repeated administration by this more pleasant route is a good substitutive of the dangerous and distressing intravitreal injection, the later non-applicable in early stages of the diseases or conditions of the posterior segment (e.g. in the dry form of AMD). Advantageously, CeO₂NPs-(C) of formula (I) did not accumulate in liver or brain at any toxic concentration.

### The sustained topical administration of cerium oxide nanoparticles does not cause cytotoxicity (one-week administration):

The effect of cerium accumulation with histological evaluation was also assessed. Retina architecture and the thickness of the different layers was evaluated. No changes were observed in mice receiving CeO₂NPs-(C) of formula (I) compared to those receiving vehicle. The thickness of all layers was also maintained between groups. Assessment of the effect of the treatment with CeO₂NPs-(C) of formula (I) in cell death was performed by TUNEL staining. Retina sections were evaluated, observing some positive cells in the outer nuclear layer (ONL) and inner nuclear layer (INL), but with no differences between eyes receiving CeO₂NPs-(C) of formula (I) or vehicle (data not shown).

### One-week daily topical treatment with CeO₂NPs does not produce reactive gliosis:

A wide range of physiological stresses and insults can induce Müller glia to undergo reactive gliosis, contributing to retinal degeneration. In order to evaluate the impact of CeO₂NPs-(C) of formula (I) accumulation in the increase of reactive gliosis, GFAP expression was analysed. CeO₂NPs administration did not increase GFAP expression in wild type retinas, observed with immunofluorescence analysis. Quantification of its expression with real-time PCR did not show any differences in its expression either (data not shown).

### CeO2NP treatment inhibits aberrant angiogenesis in vitro and in vivo:

It is known that inflammation may promote aberrant or insufficient angiogenesis. Inventors performed an assay to determine if CeO2NP treatment could inhibit any aberrant form of angiogenesis likely by decreasing inflammation, while promoting, if any, an adequate angiogenesis.

The effects of CeO₂NPs-(C) of formula (I) to avoid aberrant angiogenesis were examined using mouse choroidal sprouting assay. Choroids treated with increased concentrations of CeO₂NPs-(C) of formula (I) showed a reduction in newly formed micro-vessels length compared with choroids treated with VEGF (vascular endothelial growth factor). While VEGF promotes microvasculature to grow, CeO₂NPs-(C) of formula (I) inhibited choroid sprouting in a dose-dependent manner and better than the control (-VEGF). Data are depicted in FIG. 4, wherein micro-vessel length in micrograms is illustrated for each tested concentration and compound. Olympus microscope pictures allow also visualizing the reduction in aberrant angiogenesis by CeO₂NPs-(C) of formula (I).

The *in vivo* study of the inhibition of aberrant angiogenic activity of CeO₂NPs-(C) of formula (I) was performed in the mouse model of choroidal neovascularization induced by laser. Ocular treatment with CeO₂NPs-(C) of formula (I) during 7 days allowed observing significantly decreased neovascular lesions' areas produced by the laser as well as the activated microglia recruited by the lesion. Data of lesioned area are depicted in FIG. 5.

### EXAMPLE 2. Analysis with synthetic vitreous humour

Inventors also carried out an assay with a synthetic vitreous humour to see the distribution of the nanoparticles of the invention (CeO₂NPs-(C)) all along the liquid volume. This distribution was compared with that of another colloidal suspension including state of the art ceria nanoparticles stabilized with TMAOH (CeO₂NP-TMAOH). These later were those obtained by basic precipitation of cerium nitrate salt in water, and they were in form of aggregates of nanoparticles from 30 nm to 100 nm (including many aggregates around the 100 nm than lower than this size).

The synthetic vitreous humour (added in a tube) was not as dense as the vitreous humour of mammals, but it served for the purpose of a first approach to see how different ceria nanoparticle (that of the invention and other with aggregates) diffuse all along the different levels of the said vitreous humour.

Results can be seen in FIG. 6, where the percentage of concentration of CeO₂NP distributed along the different levels (upper U, middle M or lower L) of the synthetic vitreous humor are shown in bars. CeO₂NP were added at the upper level (as shown in figure).

Both type of nanoparticles was able to diffuse all along the vitreous humour, but this was due to the previously mentioned low density of the liquid. The main result of this assay is that with the nanoparticles of the invention (CeO₂NPs-(C) of formula (I)) a more homogeneous distribution was achieved, and in addition a higher percentage of the total nanoparticles get the lower level. These slight differences on the behaviour of the nanoparticles are of high relevance in medical conditions because they are translated into an improvement in therapy. Effectively, the therapeutic effect will not depend on the distribution of the nanoparticles. With a synthetic vitreous humour denser as the tested one, and more like that of the mammals, the differences in the distribution between the nanoparticles of formula (I) and those of the prior art (i.e., CeO₂NP-TMAOH) are likely to be increased.

### REFERENCES CITED IN THE APPLICATION

- Awwad et al., "Principles of Pharmacology in the Eye", British Journal of Pharmacology-2017, vol no. 174 Issue 23, pp.:4205-4223.
- Urtti A et al., "Challenges and obstacles of ocular pharmacokinetics and drug delivery". Adv. Drug. Deliv. Rev. 2006, vol. 58, pp. 1131-1135.
- Simó et al. on behalf of the European Consortium for Early Treatment of Diabetic Retinopathy (EUROCONDOR). "Neurodegeneration is an early event in diabetic retinopathy: therapeutic implications", Br. J. Ophthalmol. - 2012, vol. 96, pp.1285-1290
- US7347987B2
- US2011111007
- Maccarone et al., 2019. Ophtalmic Applications of Cerium Oxide Nanoparticles. Nanoceria in ophthalmology.
- Badia et al., "First steps in the development of topical CeO2NPs treatment to fight dry AMD progression using the DKOrd8 mouse model", IOVS-2018, vol. no. 59(9), Abstract 58
- Tisi et al., "Nanoceria particles are an eligible candidate to prevent age-related macular degeneration by inhibiting retinal pigment epithelium cell death and autophagy alterations", Cells-2020, vol. no. 9, p. 1617
- US2010221344
- WO2018064357
- WO2017174437
- Hancock et al., "The characterization of purified citrate-coated cerium oxide nanoparticles prepared via hydrothermal synthesis", Applied Surface Sciences-2020, 147681

## Claims

1. A single-crystal single cerium oxide nanoparticle of formula (I):
NP-(C) (I)
, wherein
NP is a single-crystal single cerium oxide nanoparticle with a crystal diameter from 3.0 to 5.0 nm, measured with transmission electron microscopy and X-Ray diffraction, and (C) is a coating of citrate molecules adsorbed on the NP,
for use in the treatment of a disorder or disease of the posterior segment of the eye, wherein the treatment comprises administration of a topical ophthalmic dose of single-crystal single cerium oxide nanoparticles of formula (I).

2. The single-crystal single cerium oxide nanoparticles of formula (I) for use according to claim 1, wherein the crystal diameter is from 3.0 to 3.5 nm, measured with transmission electron microscopy and X-Ray diffraction.

3. An aqueous colloidal suspension comprising single-crystal single cerium oxide nanoparticles of formula (I) as defined in any of claims 1-2, and a pharmaceutically acceptable citrate salt, for use in the treatment of a disorder or disease of the posterior segment of the eye, wherein the treatment comprises administration of a topical ophthalmic dose of aqueous colloidal suspension.

4. The aqueous colloidal suspension for use according to claim 3, wherein the pharmaceutically acceptable citrate salt is selected from an alkaline and alkaline-earth metal salt.

5. The aqueous colloidal suspension for use according to claim 4, wherein the pharmaceutically acceptable citrate salt is sodium citrate.

6. The aqueous colloidal suspension for use according to any of claims 3-5, wherein the topical ophthalmic dose is from 1 micrograms of cerium oxide/day to 250 micrograms of cerium oxide/day per eye.

7. The aqueous colloidal suspension for use according to any of claims 3-6, which is an eye drop colloidal suspension.

8. The single-crystal single cerium oxide nanoparticle of formula (I), or an aqueous colloidal suspension comprising said nanoparticle, for use according to any of claims 1-7, wherein the condition or disease of the posterior segment of the eye is selected from the group consisting of retinal and/or choroid pathologies, vitreous humour pathologies, posterior sclera pathology, intraocular tumors, optic nerve pathologies, and combinations thereof.

9. The single-crystal single cerium oxide nanoparticle of formula (I), or an aqueous colloidal suspension comprising said nanoparticle, for use according to any of claims 1-8, wherein the disease of the posterior segment of the eye is a retinal pathology and/or choroid pathology selected from a retinal vasculopathy, a maculopathy, an hereditary eye fundus dystrophy, an idiopathic chorioretinopathy, a central serous retinopathy, generalized choroidal dystrophy, and combinations thereof.

10. The single-crystal single cerium oxide nanoparticle of formula (I), or an aqueous colloidal suspension comprising said nanoparticle, for use according to claim 9, wherein the retinal vasculopathy is selected from diabetic retinopathy, diabetic papillopathy, non-diabetic retinopathy, ocular ischemic syndrome, hypertensive retinopathy, thalassemia retinopathy, Coats' syndrome, Eales' syndrome, radiation retinopathy, solar retinopathy, purtscher retinopathy, polypoidal choroidal vasculopathy (PCV), retinal macroaneurysm, retinal microaneurysm, leukemic retinopathy, retinal ischemia, chronic retina disorders, and combinations thereof.

11. The single-crystal single cerium oxide nanoparticle of formula (I), or an aqueous colloidal suspension comprising said nanoparticle, for use according to claim 9, wherein the maculopathy is selected from aged-related macular degeneration (AMD), hemorrhagic AMD, retinal angiomatous proliferation, polipoidal choroidal vasculopathy, malattia leventinese, full thickness macular hole, macular epiretinal membrane, macular telangiectasias, cellophane maculopathy or macular pucker, myopia maculopathy, exudative maculopathy after venous thrombosis of the retina, acute macular neuroretinopathy, macular cystoids, macular edema, retinal angioid streaks, choroidal folds, hypotony maculopathy and combinations thereof; and wherein the hereditary eye fundus distrophy is selected from the group consisting of retinitis pigmentosa; atypical retinitis pigmentosa including Usher's syndrome, retinitis punctata albicans, Leber's congenital amaurosis, dystrophy of the cones, rod dystrophy, Bietti crystalline corneoretinal dystrophy, juvenile macular dystrophy, all types of macular dystrophy, Stargardt's disease or Fundus flavimaculatus, Usher's Syndrome and combinations thereof.

12. The single-crystal single cerium oxide nanoparticle of formula (I), or an aqueous colloidal suspension comprising said nanoparticle, for use according to any of claims 1-8, wherein the disease of the posterior eye segment is a vitreous humour pathology selected from the group consisting of sub-macular and vitreous haemorrhages, asteroid hyalosis, vitreous detachment, eye floaters or myodesopsia, hereditary vitreoretinopathies, Stickler's syndrome or Wagner's Syndrome, and combinations thereof.

13. The single-crystal single cerium oxide nanoparticle of formula (I), or an aqueous colloidal suspension comprising said nanoparticle, for use according to any of claims 1-8, wherein the disease of the posterior segment of the eye is an optic nerve pathology selected from the group consisting of optic atrophy, optic neuritis, neuroretinitis, ischemic neuropathy, hereditary optic neuropathy, toxic amblyopia or nutritional optic neuropathy, ocular hypertension, primary glaucoma , secondary glaucoma, iridocorneal endothelial syndrome associated to glaucoma, head drusen or optic disc drusen, papilledema and combinations thereof.

14. A pharmaceutical or veterinary composition comprising:
(a) single-crystal single cerium oxide nanoparticles of formula (I):
NP-(C) (I)
, wherein NP is a single-crystal single cerium oxide nanoparticle with a crystal diameter from 3 to 5 nm, measured with transmission electron microscopy and X-Ray diffraction, and (C) is a coating of citrate molecules adsorbed on the NP; and
(b) a pharmaceutically or veterinary acceptable citrate salt, together with pharmaceutically or veterinary acceptable excipients and/or carriers.

15. The pharmaceutical or veterinary composition according to claim 14, which is a topical eye aqueous colloidal suspension.

## Patentansprüche

1. Ein einkristallines einzelne Ceroxid-Nanopartikel der Formel (I):
NP-(C) (I),
wobei
NP ein einkristallines einzelnes Ceroxid-Nanopartikel mit einem Kristalldurchmesser von 3,0 bis 5,0 nm, gemessen mittels Transmissionselektronenmikroskopie und Röntgenbeugung, und (C) eine Beschichtung aus am NP adsorbierten Citratmolekülen ist,
zur Verwendung bei der Behandlung einer Störung oder Erkrankung des hinteren Segments des Auges, wobei die Behandlung die Verabreichung einer topischen ophthalmischen Dosis von einkristallinen einzelnen Ceroxid-Nanopartikeln der Formel (I) umfasst.

2. Die einkristallinen einzelnen Ceroxid-Nanopartikel der Formel (I) zur Verwendung nach Anspruch 1, wobei der Kristalldurchmesser von 3,0 bis 3,5 nm beträgt, gemessen mittels Transmissionselektronenmikroskopie und Röntgenbeugung.

3. Eine wässrige kolloidale Suspension umfassend einkristalline einzelne Ceroxid-Nanopartikel der Formel (I), wie in einem der Ansprüche 1-2 definiert, und ein pharmazeutisch akzeptables Citrat-Salz, zur Verwendung bei der Behandlung einer Störung oder Erkrankung des hinteren Segments des Auges, wobei die Behandlung die Verabreichung einer topischen ophthalmischen Dosis der wässrigen kolloidalen Suspension umfasst.

4. Die wässrige kolloidale Suspension zur Verwendung nach Anspruch 3, wobei das pharmazeutisch akzeptable Citrat-Salz aus einem Alkali- und Erdalkalimetallsalz ausgewählt ist.

5. Die wässrige kolloidale Suspension zur Verwendung nach Anspruch 4, wobei das pharmazeutisch akzeptable Citrat-Salz Natriumcitrat ist.

6. Die wässrige kolloidale Suspension zur Verwendung nach einem der Ansprüche 3 bis 5, wobei die topische ophthalmische Dosis von 1 Mikrogramm Ceroxid/Tag bis 250 Mikrogramm Ceroxid/Tag pro Auge beträgt.

7. Die wässrige kolloidale Suspension zur Verwendung nach einem der Ansprüche 3 bis 6, welche eine kolloidale Augentropfensuspension ist.

8. Das einkristalline einzelne Ceroxid-Nanopartikel der Formel (I) oder eine wässrige kolloidale Suspension umfassend das Nanopartikel, zur Verwendung nach einem der Ansprüche 1 bis 7, wobei der Zustand oder die Erkrankung des hinteren Segments des Auges ausgewählt ist aus der Gruppe bestehend aus retinalen und/oder choroiden Pathologien, Glaskörperpathologien, posterioren Sklerapathologien, intraokularen Tumoren, Sehnervenpathologien und Kombinationen davon.

9. Das einkristalline einzelne Ceroxid-Nanopartikel der Formel (I) oder eine wässrige kolloidale Suspension umfassend das Nanopartikel, zur Verwendung nach einem der Ansprüche 1 bis 8, wobei die Erkrankung des hinteren Segments des Auges eine retinale Pathologie und/oder Aderhautpathologie ist, ausgewählt aus einer retinalen Vaskulopathie, einer Makulopathie, einer hereditären Augenfundusdystrophie, einer idiopathischen Chorioretinopathie, einer zentralen serösen Retinopathie, generalisierter Aderhautdystrophie und Kombinationen davon.

10. Das einkristalline einzelne Ceroxid-Nanopartikel der Formel (I) oder eine wässrige kolloidale Suspension umfassend das Nanopartikel, zur Verwendung nach Anspruch 9, wobei die retinale Vaskulopathie ausgewählt ist aus diabetischer Retinopathie, diabetischer Papillopathie, nicht-diabetischer Retinopathie, okulärem ischämischem Syndrom, hypertensiver Retinopathie, Thalassämie-Retinopathie, Coats-Syndrom, Eales-Syndrom, Strahlungsretinopathie, Retinopathia solaris, Purtscher Retinopathie, polypoidaler choroidaler Vaskulopathie (PCV), retinalem Makroaneurysma, retinalem Mikroaneurysma, leukämischer Retinopathie, retinaler Ischämie, chronischen Störungen der Retina und Kombinationen davon.

11. Das einkristalline einzelne Ceroxid-Nanopartikel der Formel (I) oder eine wässrige kolloidale Suspension umfassend das Nanopartikel, zur Verwendung nach Anspruch 9, wobei die Makulopathie ausgewählt ist aus altersbedingter Makuladegeneration (AMD), hämorrhagischer AMD, retinaler angiomatöser Proliferation, polipoidaler choroidaler Vaskulopathie, Malattia leventinese, Makulaloch voller Dicke, makulärer epiretinaler Membran, makulären Teleangiektasien, Cellophanmakulopathie oder Makulapucker, Myopiemakulopathie, exudativer Makulopathie nach venöser Thrombose der Retina, akuter makulärer Neuroretinopathie, makulären Zystoiden, Makulaödem, retinalen Angioidstreifen, Choroidalfalten, Hypotoniemakulopathie und Kombinationen davon; und wobei die hereditäre Augenfundusdistrophie ausgewählt ist aus der Gruppe bestehend aus Retinitis pigmentosa; atypischer Retinitis pigmentosa einschließlich Usher-Syndrom, Retinitis punctata albicans, Leberscher kongenitaler Amaurose, Dystrophie der Zäpfchen, Dystrophie der Stäbchen, Bietti kristallinaler korneoretinaler Dystrophie, juveniler Makuladystrophie, allen Arten von Makuladystrophien, Morbus Stargardt oder Fundus flavimaculatus, Usher-Syndrom und Kombinationen davon.

12. Das einkristalline einzelne Ceroxid-Nanopartikel der Formel (I) oder eine wässrige kolloidale Suspension umfassend das Nanopartikel, zur Verwendung nach einem der Ansprüche 1 bis 8, wobei die Erkrankung des hinteren Augensegments eine Glaskörperpathologie ist, die ausgewählt ist aus der Gruppe bestehend aus submakulären Blutungen und Glaskörperblutungen, Asteroidenhyalose, Glaskörperablösung, Augenschwimmern oder Myodesopsie, hereditären Vitreoretinopathien, dem Stickler-Syndrom oder dem Wagner-Syndrom und Kombinationen davon.

13. Das einkristalline einzelne Ceroxid-Nanopartikel der Formel (I) oder eine wässrige kolloidale Suspension umfassend das Nanopartikel, zur Verwendung nach einem der Ansprüche 1 bis 8, wobei die Erkrankung des hinteren Segments des Auges eine Sehnervenpathologie ist, die ausgewählt ist aus der Gruppe bestehend aus Optikusatrophie, Optikusneuritis, Neuroretinitis, ischämischer Neuropathie, hereditärer Optikusneuropathie, toxischer Amblyopie oder ernährungsbedingter Optikusneuropathie, okulärer Hypertonie, primärem Glaukom, sekundärem Glaukom, mit Glaukom assoziiertem iridokornealem Endothelsyndrom, Drusen der Papille oder Drusen des Sehnervs, Papillenödem und Kombinationen davon.

14. Eine pharmazeutische oder tiermedizinische Zusammensetzung umfassend:
(a) einkristalline einzelne Ceroxid-Nanopartikel der Formel (I):
NP-(C) (I),
wobei NP ein einkristallines einzelnes Ceroxid-Nanopartikel mit einem Kristalldurchmesser von 3 bis 5 nm, gemessen mittels Transmissionselektronenmikroskopie und Röntgenbeugung, und (C) eine Beschichtung aus am NP adsorbierten Citratmolekülen ist; und
(b) ein pharmazeutisch oder tiermedizinisch akzeptables Citrat-Salz zusammen mit pharmazeutisch oder tiermedizinisch akzeptablen Hilfsstoffen und/oder Trägersubstanzen.

15. Die pharmazeutische oder veterinärmedizinische Zusammensetzung nach Anspruch 14, welche eine topische wässrige kolloidale Suspension für die Augen ist.

## Revendications

1. Une nanoparticule d'oxyde de cérium unique monocristalline de formule (I) :
NP-(C) (I),
dans laquelle
NP est une nanoparticule d'oxyde de cérium unique monocristalline ayant un diamètre de cristal de 3,0 à 5,0 nm, mesuré par microscopie électronique à transmission et diffraction des rayons X, et (C) est un revêtement de molécules de citrate adsorbées sur la NP,
destinée à être utilisée dans le traitement d'un trouble ou d'une maladie du segment postérieur de l'œil, dans laquelle le traitement comprend l'administration d'une dose ophtalmique topique de nanoparticules d'oxyde de cérium uniques monocristallines de formule (I).

2. Les nanoparticules d'oxyde de cérium uniques monocristallines de formule (I) pour leur utilisation selon la revendication 1, dans lesquelles le diamètre du cristal est de 3,0 à 3,5 nm, mesuré par microscopie électronique à transmission et diffraction des rayons X.

3. Une suspension colloïdale aqueuse comprenant des nanoparticules d'oxyde de cérium uniques monocristallines de formule (I) telles que définies dans l'une quelconque des revendications 1 à 2, et un sel de citrate acceptable sur le plan pharmaceutiquement, destinée à être utilisée dans le traitement d'un trouble ou d'une maladie du segment postérieur de l'œil, dans laquelle le traitement comprend l'administration d'une dose ophtalmique topique de suspension colloïdale aqueuse.

4. La suspension colloïdale aqueuse pour l'utilisation selon la revendication 3, dans laquelle le sel de citrate acceptable sur le plan pharmaceutiquement est choisi parmi un sel de métal alcalin et alcalino-terreux.

5. La suspension colloïdale aqueuse pour l'utilisation selon la revendication 4, dans laquelle le sel de citrate acceptable sur le plan pharmaceutiquement est le citrate de sodium.

6. La suspension colloïdale aqueuse pour l'utilisation selon l'une quelconque des revendications 3 à 5, dans laquelle la dose ophtalmique topique est de 1 microgramme d'oxyde de cérium/jour à 250 microgrammes d'oxyde de cérium/jour par œil.

7. La suspension colloïdale aqueuse pour l'utilisation selon l'une quelconque des revendications 3 à 6, qui est une suspension colloïdale de gouttes ophtalmiques.

8. La nanoparticule d'oxyde de cérium unique monocristalline de formule (I), ou une suspension colloïdale aqueuse comprenant ladite nanoparticule, pour l'utilisation selon l'une quelconque des revendications 1 à 7, dans laquelle l'état ou la maladie du segment postérieur de l'œil est choisi(e) dans le groupe constitué par les pathologies rétiniennes et/ou choroïdes, les pathologies de l'humeur vitrée, la pathologie de la sclère postérieure, les tumeurs intraoculaires, les pathologies du nerf optique et des combinaisons de ceux-ci.

9. La nanoparticule d'oxyde de cérium unique monocristalline de formule (I), ou une suspension colloïdale aqueuse comprenant ladite nanoparticule, pour l'utilisation selon l'une quelconque des revendications 1 à 8, dans laquelle la maladie du segment postérieur de l'œil est une pathologie rétinienne et/ou une pathologie choroïdienne choisie parmi une vasculopathie rétinienne, une maculopathie, une dystrophie héréditaire du fond de l'œil, une choriorétinopathie idiopathique, une rétinopathie séreuse centrale, une dystrophie choroïdienne généralisée et des combinaisons de celles-ci.

10. La nanoparticule d'oxyde de cérium unique monocristalline de formule (I), ou une suspension colloïdale aqueuse comprenant ladite nanoparticule, pour l'utilisation selon la revendication 9, dans laquelle la vascularopathie rétinienne est choisie parmi la rétinopathie diabétique, la papillopathie diabétique, la rétinopathie non diabétique, le syndrome ischémique oculaire, la rétinopathie hypertensive, la rétinopathie thalassémique, le syndrome de Coats, le syndrome de Eales, la rétinopathie par rayonnement, la rétinopathie solaire, la rétinopathie de Purtscher, la vascularopathie choroïdale polypoïdale (VCP), le macroanévrisme rétinien, le microanévrisme rétinien, la rétinopathie leucémique, l'ischémie rétinienne, les troubles chroniques de la rétine, et des combinaisons de ceux-ci.

11. La nanoparticule d'oxyde de cérium unique monocristalline de formule (I), ou une suspension colloïdale aqueuse comprenant ladite nanoparticule, pour l'utilisation selon la revendication 9, dans laquelle la maculopathie est choisie parmi la dégénérescence maculaire liée à l'âge (DMLA), la DMLA hémorragique, la prolifération angiomateuse rétinienne, la vasculopathie choroïdienne polipoïdale, la malattia leventinese, le trou maculaire de pleine épaisseur, la membrane épirétinienne maculaire, les télangiectasies maculaires, la maculopathie cellophane ou le froncement maculaire, la maculopathie myopique, la maculopathie exsudative après la thrombose veineuse de la rétine, la neurorétinopathie maculaire aiguë, les cystoïdes maculaires, l'œdème maculaire, les stries angioïdes rétiniennes, les plis choroïdiens, la maculopathie hypotonique et des combinaisons de ceux-ci ; et dans laquelle la distrophie du fond de l'œil héréditaire est choisie dans le groupe constitué par la rétinite pigmentaire ; les rétinites pigmentaires atypiques, y compris le syndrome d'Usher, la rétinite ponctuée albicans, l'amaurose congénitale de Leber, la dystrophie des cônes, la dystrophie des bâtonnets, la dystrophie cornéo-rétinienne cristalline de Bietti, la dystrophie maculaire juvénile, tous les types de la dystrophie maculaire, la maladie de Stargardt ou fundus flavimaculatus, le syndrome d'Usher et des combinaisons de ceux-ci.

12. La nanoparticule d'oxyde de cérium unique monocristalline de formule (I), ou une suspension colloïdale aqueuse comprenant ladite nanoparticule, pour l'utilisation selon l'une quelconque des revendications 1 à 8, dans laquelle la maladie du segment oculaire postérieur est une pathologie de l'humour vitré choisie dans le groupe constitué par les hémorragies sous-maculaires et vitréennes, l'hyalose astéroïde, le détachement vitré, les corps flottants oculaires ou myodésopsie, les vitréorétinopathies héréditaires, le syndrome de Stickler ou le syndrome de Wagner, et des combinaisons de ceux-ci.

13. La nanoparticule d'oxyde de cérium unique monocristalline de formule (I), ou une suspension colloïdale aqueuse comprenant ladite nanoparticule, pour l'utilisation selon l'une quelconque des revendications 1 à 8, dans laquelle la maladie du segment postérieur de l'œil est une pathologie du nerf optique choisie dans le groupe constitué par l'atrophie optique, la névrite optique, la neurorétinite, la neuropathie ischémique, la neuropathie optique héréditaire, l'amblyopie toxique ou la neuropathie optique nutritionnelle, l'hypertension oculaire, le glaucome primaire, le glaucome secondaire, le syndrome endothélial iridocornéen associé au glaucome, les drusen de la tête ou du disque optique, l'œdème papillaire et des combinaisons de ceux-ci.

14. Une composition pharmaceutique ou vétérinaire comprenant :
(a) des nanoparticules d'oxyde de cérium uniques monocristallines de formule (I) :
NP-(C) (I),
dans laquelle NP est une nanoparticule d'oxyde de cérium unique monocristalline ayant un diamètre de cristal de 3 à 5 nm, mesuré par microscopie électronique à transmission et diffraction des rayons X, et (C) est un revêtement de molécules de citrate adsorbées sur la NP ; et
(b) un sel de citrate acceptable sur le plan pharmaceutique ou vétérinaire, avec des excipients et/ou des véhicules acceptables sur le plan pharmaceutique ou vétérinaire.

15. La composition pharmaceutique ou vétérinaire selon la revendication 14, qui est une suspension colloïdale aqueuse topique pour les yeux.
